# EUROPEAN PATENT SPECIFICATION

(11) **EP 3 529 360 B1**
(45) Date of publication and mention of the grant of the patent: **17.04.2024**
(21) Application number: 17800976.7
(22) Date of filing: 18.10.2017
(51) Int. Cl.: C12N 15/113, A61K 31/713

(54) **METHODS FOR PREVENTING CARDIOVASCULAR EVENTS THROUGH PROPROTEIN CONVERTASE SUBTILISIN KEXIN 9 (PCSK9) PROTEIN REDUCTION**
VERFAHREN ZUR VORBEUGUNG VON KARDIOVASKULÄREN EREIGNISSEN DURCH PROTEINREDUKTION VON PROPROTEIN-KONVERTASE-SUBTILISIN-KEXIN 9 (PCSK9)
PROCÉDÉS DE PRÉVENTION D'ÉVÉNEMENTS CARDIOVASCULAIRES PAR RÉDUCTION PROTÉIQUE DE LA PROPROTÉINE CONVERTASE SUBTILISINE KEXINE 9 (PCSK9)

(30) Priority: 18.10.2016 US 201662409816 P; 14.11.2016 US 201662422028 P; 16.03.2017 US 201762472525 P; 25.08.2017 US 201762550426 P
(43) Date of publication of application: 28.08.2019
(73) Proprietor: Novartis AG, 4056 Basel (CH)
(72) Inventor: WIJNGAARD, Peter, Parsippany NJ 07054 (US); KALLEND, David, Parsippany NJ 07054 (US)
(74) Representative: Leitner, Laura
(86) International application number: PCT/US2017/057218
(87) International publication number: WO 2018/075658

(56) References cited:
- WO-A1-2014/089313
- LADEIRAS-LOPES RICARDO ET AL: "Atherosclerosis: Recent trials, new targets and future directions", INTERNATIONAL JOURNAL OF CARDIOLOGY, ELSEVIER, AMSTERDAM, NL, vol. 192, 8 May 2015 (2015-05-08), pages 72-81, XP029174409, ISSN: 0167-5273, DOI: 10.1016/J.IJCARD.2015.05.013
- JAMES M. MCKENNEY: "Understanding PCSK9 and anti-PCSK9 therapies", JOURNAL OF CLINICAL LIPIDOLOGY, vol. 9, no. 2, 1 March 2015 (2015-03-01), pages 170-186, XP055437114, US ISSN: 1933-2874, DOI: 10.1016/j.jacl.2015.01.001

## Description

### TECHNICAL FIELD OF THE INVENTION

The present application relates to the use of an RNA interference (RNAi) agent that inhibits the synthesis of PCSK9 in a prophylactic or therapeutic methods for subjects in need thereof, wherein the RNAi agent is to be administered in a certain dosing regimen.

### BACKGROUND OF THE INVENTION

Atherosclerotic cardiovascular disease (ASCVD) remains a challenge to global health. Atherosclerosis, a systematic disease process marked by a build up of fatty deposits, inflammation cells, and scar tissue within the walls of arteries, is the underlying cause of the majority of clinical cardiovascular events. While various types of treatments and preventative measures have been developed, reducing low density lipoprotein cholesterol (LDL-C) has remained as a proven strategy for combating ASCVD. To this end, statins are a widely-used therapy for lowering lipid levels, but many at risk subjects continue to have elevated levels of LDL-C.

Proprotein convertase subtilisin kexin 9 (PCSK9) has recently been identified as a target for reducing levels of LDL-C. PCSK9 is a member of the subtilisin serine protease family and is shown to play a role in cholesterol metabolism.

WO2014089313A1 relates to RNAi agents targeting the PCSK9 gene, and methods of using such RNAi agents to inhibit expression of PCSK9.

Ladeiras-Lopes Ricardo et al, "Atherosclerosis: Recent trials, new targets and future directions", International Journal of Cardiology (20150508), vol. 192, pages 72-81 is a review of pharmacological approaches in atherosclerosis.

Monoclonal antibodies blocking PCSK9 have been developed and were demonstrated to reduce circulating PCSK9 levels and to lower LDL-C concentrations. But monoclonal antibodies of PCSK9 have a short duration of effect, which can cause significant administration, as well as financial, burdens.

Therefore, there is a need in the art for effective treatments or preventative measures aimed at reducing levels of LDL-C and, ultimately, treating or preventing ASCVD.

### SUMMARY OF THE INVENTION

The present invention provides an interfering ribonucleic acid (RNAi) agent for use in (i) the treatment of an atherosclerotic cardiovascular disease (ASCVD), an ASCVD risk equivalent, an elevated risk for cardiovascular disease (CVD), heterozygous familial hypercholesterolemia, or homozygous familial hypercholesterolemia, in a subject, or (ii) the prevention of a cardiovascular event, or the prevention of the development of an ASCVD, in a subject;
wherein the RNAi agent is a double-stranded ribonucleic acid comprising a sense strand and an antisense strand that forms a double-stranded region, the antisense strand comprising the nucleotide sequence of SEQ ID NO: 3, and the sense strand comprising the nucleotide sequence of SEQ ID NO: 4, wherein SEQ ID NO: 3 is 5'-asCfsaAfAfAfgCfaAfaAfcAfgGfuCfuagsasa-3', in which a, g, c, and u are 2'-O-methyl (2'-OMe) A, G, C, and U, respectively; Af, Gf, Cf, and Uf are 2'-fluoro A, G, C, and U, respectively; and s is a phosphorothioate linkage, and SEQ ID NO: 4 is 5'-csusagacCfuGfudTuugcuuuugu-3', in which a, g, c, and u are 2'-O-methyl (2'-OMe) A, G, C, and U, respectively; Af, Gf, Cf, and Uf are 2'-fluoro A, G, C, and U, respectively; dT is 2'-deoxythymidine; and s is a phosphorothioate linkage, and wherein the double-stranded ribonucleic acid has a covalently attached triantennary GalNAc ligand; and
wherein the RNAi agent is to be administered as a subcutaneous injection in a dosing regimen that comprises a loading phase followed by a maintenance phase, wherein the loading phase comprises administering the RNAi agent as at least two doses separated by a time interval, wherein the time interval is about 90 days.

The present invention therefore relates to the use of an RNA interference (RNAi) agent that inhibits the synthesis of PCSK9 in prophylactic or therapeutic methods for subjects in need thereof.

In one aspect thereof, the present invention relates to said RNA interference (RNAi) agent for use in a method of lowering LDL-C in a subject.

In another aspect thereof, the present invention relates to said RNA interference (RNAi) agent for use in a method of preventing a cardiovascular event in a subject.

In certain embodiments thereof, the subject may have ASCVD, ASCVD risk equivalent, an elevated risk for cardiovascular disease (CVD), heterozygous familial hypercholesterolemia, or homozygous familial hypercholesterolemia, is in need of lowering low density lipoprotein cholesterol, or otherwise has a disorder that would benefit from a reduction in LDL-C.

In a further aspect thereof, the present invention relates to said RNA interference (RNAi) agent for use in a method of preventing development of ASCVD.

In another aspect thereof, the present invention relates to said RNA interference (RNAi) agent for use in a method of treating a subject who has ASCVD, ASCVD risk equivalent, an elevated risk for CVD, heterozygous familial hypercholesterolemia, homozygous familial hypercholesterolemia, or is in need of lowering LDL-C.

In certain embodiments thereof, the methods are to treat acute coronary syndrome and/or high LDL-C levels.

In some embodiments thereof, the subject may have a baseline LDL-C greater than about 70 mg/dl, such as about 100 mg/dl.

The subject is administered multiple doses of the RNAi agent, as defined in the appended claims.

In certain embodiments thereof, the methods comprise evaluating the subject before administration of the RNAi agent. The evaluation may comprise measuring one or more biochemical parameters of the subject, including lipid parameters. Examples of parameters that may be measured before administration of the RNAi agent include levels of LDL-C, high-density lipoprotein cholesterol (HDL-C), PCSK9, total cholesterol, triglycerides, non-HDL-C, very low-density lipoprotein cholesterol (VLDL-C), apolipoprotein A1 (Apo-A1), apolipoprotein B (Apo-B), lipoprotein(a) (Lp(a)), C-reactive protein (CRP), glycated hemoglobin A1c, alanine aminotransferase, aspartate aminotransferase, alkaline aminotransferase, creatine kinase, and total bilirubin. In some embodiments, the evaluation of the subject before administration of the RNAi agent provides a baseline measurement of the biochemical parameters. In certain embodiments, the evaluation of the subject before administration of the RNAi agent determines and/or influences the administration of the RNAi agent, such as the amount of the RNAi agent, the timing of the administration of the RNAi agent, etc.

In certain embodiments thereof, the methods comprise evaluating the subject between one or more of the doses of the RNAi agent. The evaluation may comprise performing measurements on one or more biochemical parameters of the subject, including lipid parameters. Examples of parameters that may be measured between one or more of the doses of the RNAi agent include levels of LDL-C, HDL-C, PCSK9, total cholesterol, triglycerides, non-HDL-C, VLDL-C, Apo-A1, Apo-B, Lp(a), CRP, glycated hemoglobin A1c, alanine aminotransferase, aspartate aminotransferase, alkaline aminotransferase, creatine kinase, and total bilirubin. In some embodiments, the results of the evaluation may determine and/or influence the subsequent administration or subsequent administrations of the RNAi agent, such as the amount of the RNAi agent, the timing of the administration of the RNAi agent, etc.

In some embodiments thereof, administration of the RNAi agent reduces the level of LDL-C by greater than about 20 % as compared to a baseline LDL-C level. In certain embodiments, the reduction in the level of LDL-C of greater than about 20 % as compared to the baseline level is maintained for, at, or through 15 days or more after the RNAi agent is administered.

In some embodiments thereof, administration of the RNAi agent reduces the level of PCSK9 by greater than about 25 % as compared to a baseline level of PCSK9. In certain embodiments, the reduction in the level of PCSK9 of greater than about 25 % as compared to the baseline level is maintained for, at, or through 30 days or more after the RNAi agent is administered.

In some embodiments thereof, the subject may be on a background lipid-lowering therapy, such as statins. In certain embodiments, the subject is administered the RNAi agent while continuing the background lipid-lowering therapy. In certain embodiments, the subject may be on maximally tolerated statin therapy. Alternatively, the subject may be on ezetimibe or LDL apheresis.

In other embodiments thereof, the subject is not on a background lipid-lowering therapy.

In certain embodiments thereof, the subject does not have active liver disease. For instance, the subject may not exhibit a baseline level of alanine aminotransferase and/or aspartate aminotransferase that is greater than 2 times the upper limit of normal (ULN). In some embodiments, the subject may not exhibit a baseline level of total bilirubin that is greater than 1.5 times the ULN.

The present invention is further illustrated by the following detailed description and drawings.

### BRIEF DESCRIPTION OF THE DRAWINGS

Figure 1 shows an RNAi agent according to embodiments of the invention. Figure 1 discloses SEQ ID NOS: 4, 3, and 3, respectively, in order of appearance.
Figure 2 shows a summary of the dosing schedule used in the study discussed in the Example.
Figure 3 shows the mean percent change of LDL-C up to Day 360 after single administration of the RNAi agent on Day 1 as a 200-mg dose, 300-mg dose, and 500-mg dose, according to embodiments of the invention.
Figure 4 shows time-adjusted mean percent change of LDL-C up to Day 360 after single administration of the RNAi agent on Day 1 as a 200-mg dose, 300-mg dose, and 500-mg dose, and after administration of the RNAi agent on Day 1 and Day 90 as a 100-mg dose, 200-mg dose, and 300-mg dose, according to embodiments of the invention.
Figure 5 shows time-adjusted mean absolute change of LDL-C up to Day 360 after single administration of the RNAi agent on Day 1 as a 200-mg dose, 300-mg dose, and 500-mg dose, and after administration of the RNAi agent on Day 1 and Day 90 as a 100-mg dose, 200-mg dose, and 300-mg dose, according to embodiments of the invention.
Figures 6A-6B show percent change of LDL-C between baseline and Day 270 (Figure 6A), and between baseline and Day 360 (Figure 6B), of individual patients after single administration of the RNAi agent on Day 1 as a 200-mg dose, 300-mg dose, and 500-mg dose, according to embodiments of the invention.
Figure 7 shows the mean percent change of LDL-C up to Day 360 after administration of the RNAi agent on Day 1 and Day 90 as a 100-mg dose, 200-mg dose, and 300-mg dose, according to embodiments of the invention.
Figure 8 shows percent change of LDL-C between baseline and Day 180 of individual patients after administration of the RNAi agent on Day 1 and Day 90 as a 300-mg dose, according to embodiments of the invention.
Figures 9A-9B show percent change of LDL-C between baseline and Day 270 (Figure 9A), and between baseline and Day 360 (Figure 9B), of individual patients after administration of the RNAi agent on Day 1 and Day 90 as a 100-mg dose, 200-mg dose, and 300-mg dose, according to embodiments of the invention.

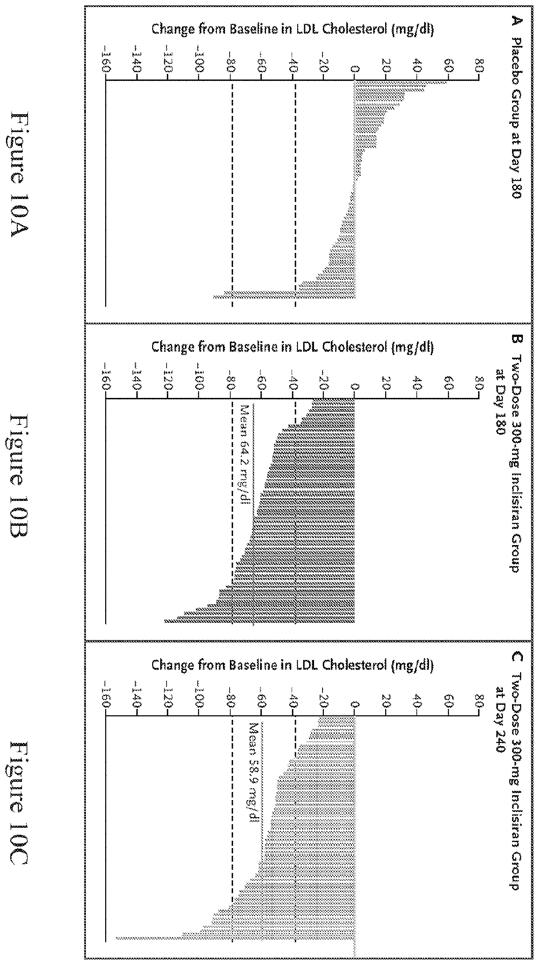

Figures 10A-10C show the change in LDL-C from baseline to Day 180 for each patient randomly assigned to the two-dose placebo group (61 patients) (Figure 10A); the change in LDL-C from baseline to Day 180 for each patient randomly assigned to the two-dose 300-mg RNAi agent (59 patients) (Figure 10B); and the change in LDL-C from baseline to Day 240 for each patient randomly assigned to and the two-dose 300-mg RNAi agent group (59 patients) (Figure 10C), according to embodiments of the invention. Dashed lines represent LDL-C reductions of 39 mg per deciliter and 78 mg per deciliter (to convert the values for cholesterol to millimoles per liter, multiply by 0.02586).
Figures 11A-11B shows percent change of LDL-C from baseline to Day 90 and Day 270 of individual patients after administration of the RNAi agent on Day 1 as a 300-mg dose (Figure 11A), or after administration of the RNAi agent on Day 1 and Day 90 as a 300-mg dose (Figure 11B), according to embodiments of the invention.
Figure 12 shows the mean percent change of PCSK9 levels up to Day 270 after single administration of the RNAi agent on Day 1 as a 200-mg dose, 300-mg dose, and 500-mg dose, according to embodiments of the invention.
Figure 13 shows the mean percent change of PCSK9 levels up to Day 270 after administration of the RNAi agent on Day 1 and Day 90 as a 100-mg dose, 200-mg dose, and 300-mg dose, according to embodiments of the invention.
Figure 14 shows modeled results of LDL-C up to 22 months after administration of the RNAi agent administered twice or thrice annually as a 300-mg dose, according to embodiments of the invention.

### DETAILED DESCRIPTION OF THE INVENTION

The present invention is based, at least in part, on the use of small interfering RNA (siRNA) molecules as a means to reduce levels of PCSK9 protein, which in turn lowers LDL-C levels. Through RNA interference, the siRNA bind intracellularly to the RNA-induced silencing complex (RISC), which enables it to cleave messenger RNA (mRNA) that encodes PCSK9. The cleaved mRNA is degraded and therefore unavailable for protein translation, resulting in decreased levels of the PSCK9 protein and, consequently, decreased levels of LDL-C.

Accordingly, the present invention provides an interfering ribonucleic acid (RNAi) agent for use in (i) the treatment of an atherosclerotic cardiovascular disease (ASCVD), an ASCVD risk equivalent, an elevated risk for cardiovascular disease (CVD), heterozygous familial hypercholesterolemia, or homozygous familial hypercholesterolemia, in a subject, or (ii) the prevention of a cardiovascular event, or the prevention of the development of an ASCVD, in a subj ect;
wherein the RNAi agent is a double-stranded ribonucleic acid comprising a sense strand and an antisense strand that forms a double-stranded region, the antisense strand comprising the nucleotide sequence of SEQ ID NO: 3, and the sense strand comprising the nucleotide sequence of SEQ ID NO: 4, wherein SEQ ID NO: 3 is 5'-asCfsaAfAfAfgCfaAfaAfcAfgGfuCfuagsasa-3', in which a, g, c, and u are 2'-O-methyl (2'-OMe) A, G, C, and U, respectively; Af, Gf, Cf, and Uf are 2'-fluoro A, G, C, and U, respectively; and s is a phosphorothioate linkage, and SEQ ID NO: 4 is 5'-csusagacCfuGfudTuugcuuuugu-3', in which a, g, c, and u are 2'-O-methyl (2'-OMe) A, G, C, and U, respectively; Af, Gf, Cf, and Uf are 2'-fluoro A, G, C, and U, respectively; dT is 2'-deoxythymidine; and s is a phosphorothioate linkage, and wherein the double-stranded ribonucleic acid has a covalently attached triantennary GalNAc ligand; and
wherein the RNAi agent is to be administered as a subcutaneous injection in a dosing regimen that comprises a loading phase followed by a maintenance phase, wherein the loading phase comprises administering the RNAi agent as at least two doses separated by a time interval, wherein the time interval is about 90 days.

Accordingly, in one aspect of the present invention, said RNAi agent is for use in a method of lowering LDL-C in a subject, the method comprising administering to the subject an effective amount, such as a prophylactically-effective amount or a therapeutically-effective amount, of said RNAi agent. In one aspect, the present invention also provides said RNAi agent for use in a method of preventing a cardiovascular event in a subject, the method comprising administering to the subject an effective amount, such as a prophylactically-effective amount or a therapeutically-effective amount, of said RNAi agent. For these methods, the subject may have ASCVD, ASCVD risk equivalent, an elevated risk for CVD, heterozygous familial hypercholesterolemia, homozygous familial hypercholesterolemia, or is in need of lowering LDL-C.

Further, in one aspect, the present invention provides said RNAi agent for use in a method of preventing development of ASCVD in a subject, the method comprising administering to the subject an effective amount, such as a prophylactically-effective amount or a therapeutically-effective amount, of an RNAi agent.

In addition, in one aspect, the present invention provides said RNAi agent for use in a method of treating a subject who has ASCVD, ASCVD risk equivalent, an elevated risk for CVD, heterozygous familial hypercholesterolemia, homozygous familial hypercholesterolemia, or is in need of lowering LDL-C. The method may comprise administering to the subject an effective amount, such as a prophylactically-effective amount or a therapeutically-effective amount, of an RNAi agent.

Moreover, in one aspect, the present invention relates to said RNAi agent for use in the methods described herein, i.e., for use in lowering LDL-C in a subject; preventing a cardiovascular event in a subject; preventing development of ASCVD in a subject; and/or treating a subject who has ASCVD, ASCVD risk equivalent, an elevated risk for CVD, heterozygous familial hypercholesterolemia, homozygous familial hypercholesterolemia, or is in need of lowering LDL-C.

In some embodiments of the present invention, the cardiovascular event may be a major adverse cardiovascular event, which includes, but is not limited to death, nonfatal myocardial infarction, severe recurrent ischemia, stroke, symptomatic pulmonary embolism, and bleeding.

In some embodiments, said RNAi agent is for use as described herein, wherein the method of lowering LDL-C and/or preventing a cardiovascular event may be directed to subjects who have ASCVD or who are "ASCVD risk equivalent." In certain embodiments, a subject may be "ASCVD risk equivalent" if he/she has one or more of the following: symptomatic atherosclerosis, Type 2 diabetes, familial hypercholesterolemia, including subjects whose 10-year risk of a cardiovascular event assessed by Framingham Risk Score (score > 20 %) or equivalent has a target LDL-C of < 100 mg/dl. In some embodiments, the subject may have heterozygous familial hypercholesterolemia.

In some embodiments, said RNAi agent is for use as described herein, wherein the method of lowering LDL-C and/or preventing a cardiovascular event also may be directed to subjects who present one or more symptoms or risk factors for having ASCVD, of being "ASCVD risk equivalent," or otherwise having a potential to develop cardiac health issues. Such symptoms/risk factors may include having acute coronary syndromes; having a history of myocardial infarction, stable or unstable angina, coronary or other arterial revascularization, stroke, transient ischemic attack, or peripheral arterial disease of atherosclerotic origin; being male; having a family history of heart disease, ASCVD, or ASCVD risk equivalent; having a smoking habit; being physically inactive; having high blood pressure; having high blood cholesterol; having diabetes or prediabetes; being overweight or obese; having a history of preeclampsia during pregnancy; having uncontrolled stress and/or anger; being post-menopausal; having an unhealthy diet, e.g., a diet high in salt, saturated fat, trans fat, cholesterol, and/or refined sugars; being age 55 or older; having sleep apnea; having anemia; or a combination thereof. Therefore, the method can be directed to, for example, subjects who are young but have a familial history of heart disease, or subjects who smoke but do not have high blood pressure. In certain embodiments, the method may be directed to subjects who previously experienced a cardiac event.

In some embodiments, said RNAi agent is for use as described herein, wherein the method of the invention may be of lowering LDL-C and/or preventing a cardiovascular event in a subject having heterozygous familial hypercholesterolemia, the method comprising administering an effective amount, such as a prophylactically-effective amount or a therapeutically-effective amount, of an RNAi agent. In some embodiments, said RNAi agent is for use as described herein, wherein the method of the invention may be of lowering LDL-C and/or preventing a cardiovascular event in a subject having ASCVD, the method comprising administering an effective amount, such as a prophylactically-effective amount or a therapeutically-effective amount, of said RNAi agent. In some embodiments, said RNAi agent is for use as described herein, wherein the method of the invention may be of lowering LDL-C and/or preventing a cardiovascular event in a subject having ASCVD risk equivalent, the method comprising administering an effective amount, such as a prophylactically-effective amount or a therapeutically-effective amount, of said RNAi agent. In some embodiments, said RNAi agent is for use as described herein, wherein the method of the invention may be of lowering LDL-C and/or preventing a cardiovascular event in a subject having homozygous familial hypercholesterolemia, the method comprising administering an effective amount, such as a prophylactically-effective amount or a therapeutically-effective amount, of said RNAi agent. In some embodiments, said RNAi agent is for use as described herein, wherein the method of the invention may be of treating a subject having heterozygous familial hypercholesterolemia, the method comprising administering an effective amount, such as a prophylactically-effective amount or a therapeutically-effective amount, of said RNAi agent. In some embodiments, said RNAi agent is for use as described herein, wherein the method of the invention may be of treating a subject having ASCVD, the method comprising administering an effective amount, such as a prophylactically-effective amount or a therapeutically-effective amount, of said RNAi agent. In some embodiments, said RNAi agent is for use as described herein, wherein the method of the invention may be of treating a subject having ASCVD risk equivalent, the method comprising administering an effective amount, such as a prophylactically-effective amount or a therapeutically-effective amount, of said RNAi agent. In some embodiments, said RNAi agent is for use as described herein, wherein the method of the invention may be of treating a subject having homozygous familial hypercholesterolemia, the method comprising administering an effective amount, such as a prophylactically-effective amount or a therapeutically-effective amount, of said RNAi agent. In these embodiments, said RNAi agent is a double-stranded ribonucleic acid comprising a sense strand and an antisense strand that forms a double-stranded region, in which the antisense strand comprises the nucleotide sequence of SEQ ID NO: 3, and the sense strand comprises the nucleotide sequence of SEQ ID NO: 4, as defined in the appended claims. The subjects may be on a diet designed to improve lipid levels, and/or may be on an LDL-lowering therapy, such as a statin, ezetimibe, or LDL apheresis. In some embodiments, the subject may be on a maximally tolerated statin therapy. Further, the subjects may require additional lowering of LDL-C.

In certain embodiments, administration of the RNAi agent may reduce the level of LDL-C, as compared to a baseline LDL-C level, by greater than about 20 %, or by greater than about 25 %, or by greater than about 30 %, or by greater than about 35 %, or by greater than about 40 %, or by greater than about 45 %, or by greater than about 50 %, or by greater than about 55 %, or by greater than about 60 %.

In some embodiments, the reduction in the level of LDL-C after the RNAi agent is administered may be maintained for, at, or through about 15 days or more, or about 20 days or more, or about 30 days or more, or about 40 days or more, or about 50 days or more, or about 60 days or more, or about 70 days or more, or about 80 days or more, or about 90 days or more, or about 100 days or more, or about 110 days or more, or about 120 days or more, or about 130 days or more, or about 140 days or more, or about 150 days or more, or about 160 days or more, or about 170 days or more, or about 180 days or more, or about 190 days or more, or about 200 days or more, or about 210 days or more, or about 220 days or more, or about 230 days or more, or about 240 days or more, or about 250 days or more, or about 260 days or more, or about 270 days or more, or about 280 days or more, or about 290 days or more, or about 300 days or more, or about 310 days or more, or about 320 days or more, or about 330 days or more, or about 340 days or more, or about 350 days or more, or about 360 days or more, after the administration of the RNAi agent.

Administration of the RNAi agent in a dose amount of about 100 mg may reduce the level of LDL-C, as compared to a baseline LDL-C level, by over about 15 %, or by over about 20 %, by Day 15. This reduction of LDL-C may be maintained for, at, or through about 30 days, or about 60 days, or about 90 days, or longer. In some embodiments, the reduction of LDL-C, as compared to a baseline LDL-C level, may be greater than about 40 % at Day 30 and maintained for, at, or through about 60 days, about 90 days, or longer.

Administration of the RNAi agent in a dose amount of about 200 mg may reduce the level of LDL-C, as compared to a baseline LDL-C level, by over about 15 %, or by over about 20 %, or by over about 25 %, or by over about 30 %, by Day 15. This reduction of LDL-C may be maintained for, at, or through about 30 days, or about 60 days, or about 90 days, or about 120 days, or about 150 days, or about 180 days, or about 210 days, or about 240 days, or about 270 days, or about 300 days, or about 330 days, or about 360 days, or longer. In some embodiments, the reduction of LDL-C, as compared to a baseline LDL-C level, may be greater than about 40 % at Day 30 and maintained for, at, or through about 60 days, about 90 days, or longer. In certain embodiments, the reduction of LDL-C, as compared to a baseline LDL-C level, may be greater than about 30 % at Day 15 and maintained for, at, or through about 30 days, about 60 days, about 90 days, about 120 days, about 150 days, or longer. In some embodiments, the reduction of LDL-C, as compared to a baseline LDL-C level, may be greater than about 5 % at or through Day 180. In certain embodiments, the reduction of LDL-C, as compared to a baseline LDL-C level, may be greater than about 20 % at Day 15 and maintained for, at, or through about 30 days, or about 60 days, or about 90 days, or about 120 days, or about 150 days, or about 180 days, or about 210 days, or about 240 days, or about 270 days, or about 300 days, or about 330 days, or about 360 days. In certain embodiments, the reduction of LDL-C, as compared to a baseline LDL-C level, may be greater than about 25 % at Day 180, or greater than about 25 % at Day 270, or greater than about 30 % at Day 360.

Administration of the RNAi agent in a dose amount of about 300 mg may reduce the level of LDL-C, as compared to a baseline LDL-C level, by over about 15 %, or by over about 20 %, or by over about 25 %, or by over about 30 %, or by over about 40 % by Day 15. This reduction of LDL-C may be maintained for, at, or through about 30 days, or about 60 days, or about 90 days, or about 120 days, or about 150 days, or about 180 days, or about 210 days, or about 240 days, or about 270 days, or about 300 days, or about 330 days, or about 360 days, or longer. In some embodiments, the reduction of LDL-C, as compared to a baseline LDL-C level, may be greater than about 45 % at Day 30 and maintained for, at, or through about 60 days, about 90 days, or longer. In certain embodiments, the reduction of LDL-C, as compared to a baseline LDL-C level, may be greater than about 40 % at Day 15 and maintained for, at, or through about 30 days, about 60 days, about 90 days, about 120 days, about 150 days, or longer. In some embodiments, the reduction of LDL-C, as compared to a baseline LDL-C level, may be greater than about 35 % at Day 15, and maintained for, at, or through about 30 days, or about 60 days, or about 90 days, or about 120 days, or about 150 days, or about 180 days, or about 210 days, or longer. In some embodiments, the reduction of LDL-C, as compared to a baseline LDL-C level, may be greater than about 30 % at Day 15, and maintained for, at, or through about 30 days, or about 60 days, or about 90 days, or about 120 days, or about 150 days, or about 180 days, or about 210 days, or about 240 days, or about 270 days, or about 300 days, or about 330 days, or about 360 days or longer. In certain embodiments, the reduction of LDL-C, as compared to a baseline LDL-C level, may be greater than about 35 % at Day 180, or greater than about 30 % at Day 270, or greater than about 30 % at Day 360.

Administration of the RNAi agent in a dose amount of about 500 mg may reduce the level of LDL-C, as compared to a baseline LDL-C level, by over about 15 %, or by over about 20 %, or by over about 25 %, or by over about 30 %, or by over about 40 % by Day 15. This reduction of LDL-C may be maintained for, at, or through about 30 days, or about 60 days, or about 90 days, or about 120 days, or about 150 days, or about 180 days, or about 210 days, or about 240 days, or about 270 days, or about 300 days, or about 330 days, or about 360 days, or longer. In some embodiments, the reduction of LDL-C, as compared to a baseline LDL-C level, may be greater than about 50 % at Day 30 and maintained for, at, or through about 60 days, about 90 days, or longer. In certain embodiments, the reduction of LDL-C, as compared to a baseline LDL-C level, may be greater than about 40 % at Day 15 and maintained for, at, or through about 30 days, about 60 days, about 90 days, about 120 days, about 150 days, about 180 days, or longer. In some embodiments, the reduction of LDL-C, as compared to a baseline LDL-C level, may be greater than about 35 % at Day 15, and maintained for, at, or through about 30 days, or about 60 days, or about 90 days, or about 120 days, or about 150 days, or about 180 days, or about 210 days, or about 240 days, or longer. In some embodiments, the reduction of LDL-C, as compared to a baseline LDL-C level, may be greater than about 30 % at Day 15, and maintained for, at, or through about 30 days, or about 60 days, or about 90 days, or about 120 days, or about 150 days, or about 180 days, or about 210 days, or about 240 days, or about 270 days, or about 300 days, or longer. In certain embodiments, the reduction of LDL-C, as compared to a baseline LDL-C level, may be greater than about 40 % at Day 180, or greater than about 30 % at Day 270, or greater than about 30 % at Day 360.

Administration of a second dose of RNAi agent at Day 90 may further reduce the level of LDL-C as compared to a baseline LDL-C level. For example, after administration of about 100 mg dose of RNAi agent on Day 1, administration of about 100 mg dose of RNAi agent on Day 90 may reduce the level of LDL-C, as compared to a baseline LDL-C level, by over about 40 % on Day 104 through Day 120 and/or Day 150; by over about 35 % on Day 104 through Day 120, Day 150, and/or Day 180; by over about 30 % on Day 104 through Day 120, Day 150, Day 180, and/or Day 210; by over about 25 % on Day 104 through Day 120, Day 150, Day 180, Day 210, Day 240, and/or Day 270; by over about 20 % on Day 104 through Day 120, Day 150, Day 180, Day 210, Day 240, Day 270, Day 300, and/or Day 330; and/or by over about 10 % on Day 104 through Day 120, Day 150, Day 180, Day 210, Day 240, Day 270, Day 300, Day 330, and/or Day 360. In certain embodiments, the reduction of LDL-C, as compared to a baseline LDL-C level, may be greater than about 35 % at Day 180, or greater than about 25 % at Day 270, or greater than about 10 % at Day 360.

After administration of about 200 mg dose of RNAi agent on Day 1, administration of about 200 mg dose of RNAi agent on Day 90 may reduce the level of LDL-C, as compared to a baseline LDL-C level, by over about 45 % on Day 104 through Day 120 and/or Day 150; by over about 40 % on Day 104 through Day 120, Day 150, Day 180, Day 210, and/or Day 240; by over about 35 % on Day 104 through Day 120, Day 150, Day 180, Day 210, Day 240, Day 270, and/or Day 300; and/or by over about 30 % or less on Day 104 through Day 120, Day 150, Day 180, Day 210, Day 240, Day 270, Day 300, Day 330, and/or Day 360. In certain embodiments, the reduction of LDL-C, as compared to a baseline LDL-C level, may be greater than about 40 % at Day 180, or greater than about 35 % at Day 270, or greater than about 30 % at Day 360.

After administration of about 300 mg dose of RNAi agent on Day 1, administration of about 300 mg dose of RNAi agent on Day 90 may reduce the level of LDL-C, as compared to a baseline LDL-C level, by over about 50 % on Day 104 through Day 120, Day 150, Day 180, and/or Day 210; by over about 40 % on Day 104 through Day 120, Day 150, Day 180, Day 210, Day 240, Day 270, and/or Day 300; by over about 35 % on Day 104 through Day 120, Day 150, Day 180, Day 210, Day 240, Day 270, Day 300, and/or Day 310; and/or by over about 30 % or less on Day 104 through Day 120, Day 150, Day 180, Day 210, Day 240, Day 270, Day 300, Day 330, and/or Day 360. In certain embodiments, the reduction of LDL-C, as compared to a baseline LDL-C level, may be greater than about 50 % at Day 180, or greater than about 40 % at Day 270, or greater than about 30 % at Day 360.

In certain embodiments, administration of the RNAi agent may reduce the level of PCSK9, as compared to a baseline PCSK9 level, by greater than about 25 %, or by greater than about 30 %, or by greater than about 35 %, or by greater than about 40 %, or by greater than about 45 %, or by greater than about 50 %, or by greater than about 55 %, or by greater than about 60 %, or by greater than about 65 %, or by greater than about 70 %, or by greater than about 75 %, or by greater than about 80 %, or by greater than about 85 %, or by greater than about 90 %, or by greater than about 95 %.

In some embodiments, the reduction in the level of PCSK9 may be maintained after the RNAi agent is administered for, at, or through about 15 days or more, or about 20 days or more, or about 30 days or more, or about 40 days or more, or about 50 days or more, or about 60 days or more, or about 70 days or more, or about 80 days or more, or about 90 days or more, or about 100 days or more, or about 110 days or more, or about 120 days or more, or about 130 days or more, or about 140 days or more, or about 150 days or more, or about 160 days or more, or about 170 days or more, or about 180 days or more, or about 190 days or more, or about 200 days or more, or about 210 days or more, or about 220 days or more, or about 230 days or more, or about 240 days or more, or about 250 days or more, or about 260 days or more, or about 270 days or more, or about 280 days or more, or about 290 days or more, or about 300 days or more, or about 310 days or more, or about 320 days or more, or about 330 days or more, or about 340 days or more, or about 350 days or more, or about 360 days or more.

Administration of the RNAi agent in a dose amount of about 100 mg may reduce the level of PCSK9, as compared to a baseline PCSK9 level, by over about 20 %, or by over about 25 %, or by over about 35 %, or by over about 40 %, by Day 15. This reduction of PCSK9 may be maintained for, at, or through about 30 days, or about 60 days, or about 90 days, or longer.

Administration of the RNAi agent in a dose amount of 200 mg may reduce the level of PCSK9, as compared to a baseline PCSK9 level, by over about 20 %, or by over about 25 %, or by over about 30 %, or by over about 35 %, or by over about 40 %, or by over about 45 %, or by over about 50 %, by Day 15. This reduction of PCSK9 may be maintained for, at, or through about 30 days, or about 60 days, or about 90 days, or longer. In some embodiments, the reduction of PCSK9, as compared to a baseline PCSK9 level, may be greater than about 60 % at Day 30 and maintained for, at, or through about 60 days, about 90 days, or longer. In certain embodiments, the reduction of PCSK9, as compared to a baseline PCSK9 level, may be greater than about 30 % through Day 300.

Administration of the RNAi agent in a dose amount of about 300 mg may reduce the level of PCSK9, as compared to a baseline PCSK9 level, by over about 20 %, or by over about 25 %, or by over about 30 %, or by over about 35 %, or by over about 40 %, or by over about 45 %, or by over about 50 %, or by over about 55 %, or by over about 60 %, by Day 15. This reduction of PCSK9 may be maintained for, at, or through about 30 days, or about 60 days, or about 90 days, or longer. In some embodiments, the reduction of PCSK9, as compared to a baseline PCSK9 level, may be greater than about 60 % at Day 30 and maintained for, at, or through about 60 days, about 90 days, or longer. In certain embodiments, the reduction of PCSK9, as compared to a baseline PCSK9 level, may be greater than about 30 % through Day 300.

Administration of the RNAi agent in a dose amount of about 500 mg may reduce the level of PCSK9, as compared to a baseline PCSK9 level, by over about 20 %, or by over about 25 %, or by over about 30 %, or by over about 35 %, or by over about 40 %, or by over about 45 %, or by over about 50 %, or by over about 55 %, or by over about 60 %, by Day 15. This reduction of PCSK9 may be maintained for, at, or through about 30 days, or about 60 days, or about 90 days, or longer. In some embodiments, the reduction of PCSK9, as compared to a baseline PCSK9 level, may be greater than about 70 % at Day 30 and maintained for, at, or through about 60 days, about 90 days, or longer. In some embodiments, the reduction of PCSK9, as compared to a baseline PCSK9 level, may be greater than about 30 % through Day 300.

Administration of about 100 mg dose of RNAi agent on Day 1 followed by administration of about 100 mg dose of RNAi agent on Day 90 may reduce the level of PCSK9, as compared to a baseline PCSK9 level, by over about 20 %, or by over about 25 %, or by over about 30 %, or by over about 35 %, or by over about 40 %, by Day 104. This reduction of PCSK9 may be maintained for, at, or through about 120 days. In some embodiments, the reduction of PCSK9, as compared to a baseline PCSK9 level, may be greater than about 30 % at Day 104 and maintained for, at, or through about 150 days.

Administration of about 200 mg dose of RNAi agent on Day 1 followed by administration of about 200 mg dose of RNAi agent on Day 90 may reduce the level of PCSK9, as compared to a baseline PCSK9 level, by over about 20 %, or by over about 25 %, or by over about 30 %, or by over about 35 %, or by over about 40 %, or by over 45 %, or by over 50 % by Day 104. This reduction of PCSK9 may be maintained for, at, or through about 120 days. In some embodiments, the reduction of PCSK9, as compared to a baseline PCSK9 level, may be greater than about 40 % at Day 104 and maintained for, at, or through about 150 days.

Administration of about 300 mg dose of RNAi agent on Day 1 followed by administration of about 300 mg dose of RNAi agent on Day 90 may reduce the level of PCSK9, as compared to a baseline PCSK9 level, by over about 20 %, or by over about 25 %, or by over about 30 %, or by over about 35 %, or by over about 40 %, or by over 45 %, or by over 50 % by Day 104. This reduction of PCSK9 may be maintained for, at, or through about 120 days or through 150 days. In some embodiments, the reduction of PCSK9, as compared to a baseline PCSK9 level, may be greater than about 50 %, or greater than about 55 % at Day 120 and maintained for, at, or through about 150 days.

Administration of the RNAi agent in a dose amount of about 100 mg may reduce the level of total cholesterol, as compared to a baseline total cholesterol level, by over about 5 %, or by over about 10 %, or by over about 15 %, or by over about 20 %, by Day 90; may reduce the level of non-HDL-C, as compared to a baseline non-HDL-C level, by over about 5 %, or by over about 10 %, or by over about 15 %, or by over about 20 %, or by over about 25 %, or by over about 30 %, by Day 90; may reduce the level of Apo-B, as compared to a baseline Apo-B level, by over about 5 %, or by over about 10 %, or by over about 15 %, or by over about 20 %, or by over about 25%, by Day 90; and/or may reduce the level of Lp(a), as compared to a baseline Lp(a) level, by over about 5 %, or by over about 10 %, or by over about 15 %, by Day 90.

Administration of the RNAi agent in a dose amount of about 200 mg may reduce the level of total cholesterol, as compared to a baseline total cholesterol level, by over about 5 %, or by over about 10 %, or by over about 15 %, or by over about 20 %, or by over about 25 %, by Day 90; may reduce the level of non-HDL-C, as compared to a baseline non-HDL-C level, by over about 5 %, or by over about 10 %, or by over about 15 %, or by over about 20 %, or by over about 25%, or by over about 30%, or by over about 35 %, by Day 90; may reduce the level of Apo-B, as compared to a baseline Apo-B level, by over about 5 %, or by over about 10 %, or by over about 15 %, or by over about 20 %, or by over about 25%, or by over about 30 %, by Day 90; and/or may reduce the level of Lp(a), as compared to a baseline Lp(a) level, by over about 5 %, or by over about 10 %, or by over about 15 %, or by over about 20 %, by Day 90. In some embodiments, at Day 180, the reduction of total cholesterol is over about 15 %, non-HDL-C is over about 25 %, and Apo-B is over about 20 %.

Administration of the RNAi agent in a dose amount of about 300 mg may reduce the level of total cholesterol, as compared to a baseline total cholesterol level, by over about 5 %, or by over about 10 %, or by over about 15 %, or by over about 20 %, or by over about 25 %, by Day 90; may reduce the level of non-HDL-C, as compared to a baseline non-HDL-C level, by over about 5 %, or by over about 10 %, or by over about 15 %, or by over about 20 %, or by over about 25%, or by over about 30%, or by over about 35 %, or by over about 40 %, by Day 90; may reduce the level of Apo-B, as compared to a baseline Apo-B level, by over about 5 %, or by over about 10 %, or by over about 15 %, or by over about 20 %, or by over about 25%, or by over about 30 %, or by over about 35 %, by Day 90; and/or may reduce the level of Lp(a), as compared to a baseline Lp(a) level, by over about 5 %, or by over about 10 %, or by over about 15 %, or by over about 20 %, by Day 90.

Administration of the RNAi agent in a dose amount of about 500 mg may reduce the level of total cholesterol, as compared to a baseline total cholesterol level, by over about 5 %, or by over about 10 %, or by over about 15 %, or by over about 20 %, or by over about 25 %, or by over about 30 %, by Day 90; may reduce the level of non-HDL-C, as compared to a baseline non-HDL-C level, by over about 5 %, or by over about 10 %, or by over about 15 %, or by over about 20 %, or by over about 25%, or by over about 30%, or by over about 35 %, or by over about 40 %, by Day 90; may reduce the level of Apo-B, as compared to a baseline Apo-B level, by over about 5 %, or by over about 10 %, or by over about 15 %, or by over about 20 %, or by over about 25%, or by over about 30 %, or by over about 35 %, or by over about 40 %, by Day 90; and/or may reduce the level of Lp(a), as compared to a baseline Lp(a) level, by over about 5 %, or by over about 10 %, or by over about 15 %, or by over about 20 %, by Day 90. In some embodiments, at Day 180, the reduction of total cholesterol is over about 25 % and Apo-B is over about 30 %.

Administration of about 100 mg dose of RNAi agent on Day 1 followed by administration of about 100 mg dose of RNAi agent on Day 90 may reduce the level of total cholesterol, as compared to a baseline total cholesterol level, by over about 5 %, or by over about 10 %, or by over about 15 %, or by over about 20 %, by Day 180; may reduce the level of non-HDL-C, as compared to a baseline non-HDL-C level, by over about 5 %, or by over about 10 %, or by over about 15 %, or by over about 20 %, or by over about 25 %, or by over about 30 %, by Day 180; may reduce the level of Apo-B, as compared to a baseline Apo-B level, by over about 5 %, or by over about 10 %, or by over about 15 %, or by over about 20 %, or by over about 25%, by Day 180; and/or may reduce the level of Lp(a), as compared to a baseline Lp(a) level, by over about 5 %, or by over about 10 %, by Day 180.

Administration of about 200 mg dose of RNAi agent on Day 1 followed by administration of about 200 mg dose of RNAi agent on Day 90 may reduce the level of total cholesterol, as compared to a baseline total cholesterol level, by over about 5 %, or by over about 10 %, or by over about 15 %, or by over about 20 %, or by over about 25 %, by Day 180; may reduce the level of non-HDL-C, as compared to a baseline non-HDL-C level, by over about 5 %, or by over about 10 %, or by over about 15 %, or by over about 20 %, or by over about 25 %, or by over about 30 %, or by over 35 %, by Day 180; may reduce the level of Apo-B, as compared to a baseline Apo-B level, by over about 5 %, or by over about 10 %, or by over about 15 %, or by over about 20 %, or by over about 25%, or by over 30 %, or by over 35 %, by Day 180; and/or may reduce the level of Lp(a), as compared to a baseline Lp(a) level, by over about 5 %, or by over about 10 %, or by over 15 %, by Day 180.

Administration of about 300 mg dose of RNAi agent on Day 1 followed by administration of about 300 mg dose of RNAi agent on Day 90 may reduce the level of total cholesterol, as compared to a baseline total cholesterol level, by over about 5 %, or by over about 10 %, or by over about 15 %, or by over about 20 %, or by over about 25 %, or by over 30 %, by Day 180; may reduce the level of non-HDL-C, as compared to a baseline non-HDL-C level, by over about 5 %, or by over about 10 %, or by over about 15 %, or by over about 20 %, or by over about 25 %, or by over about 30 %, or by over 35 %, or by over 40 %, or by over 45 %, by Day 180; may reduce the level of Apo-B, as compared to a baseline Apo-B level, by over about 5 %, or by over about 10 %, or by over about 15 %, or by over about 20 %, or by over about 25%, or by over 30 %, or by over 35 %, or by over 40 %, by Day 180; and/or may reduce the level of Lp(a), as compared to a baseline Lp(a) level, by over about 5 %, or by over about 10 %, or by over 15 %, or by over 20 %, or by over 25 %, by Day 180.

In certain embodiments, said RNAi agent is for use as described herein, wherein the methods may further comprise evaluating the subject before administration of the RNAi agent. The evaluation may be performed on the same day but before the administration of the RNAi agent, or it may be performed one day before, or two days before, or three days before, or five days before, or six days before, or seven days before, or eight days before, or nine days before, or ten days before, or 11 days before, or 12 days before, or 13 days before, or 14 days before. In some embodiments, the evaluation may be performed across more than one day before the administration of the RNAi agent.

In some embodiments, the evaluation may comprise measuring one or more physiological parameters or characteristics of the subject, including but not limited to age, height, weight, body mass index, race, gender, whether the subject is undergoing any other treatment (e.g., lipid-lowering therapy such as a statin), diagnosis of cardiovascular disease, and diagnosis of diabetes mellitus, heart rate, blood pressure, electrocardiogram parameters, etc.

In certain embodiments, the evaluation may comprise measuring one or more biochemical parameters of the subjects. In some embodiments, the measurements taken prior to administration of the RNAi agent may be considered as "baseline" measurements. Examples of biochemical parameters, which include lipid parameters, may be, but are not limited to, levels of LDL-C, HDL-C, PCSK9, total cholesterol, triglycerides, non-HDL-C, VLDL-C, Apo-A1, Apo-B, Lp(a), CRP, glycated hemoglobin A1c, alanine aminotransferase, aspartate aminotransferase, alkaline aminotransferase, creatine kinase, and total bilirubin. In some embodiments, the evaluation of the subject before administration of the RNAi agent may provide a baseline measurement of the biochemical parameters. In certain embodiments, the evaluation of the subject before administration of the RNAi agent may determine and/or influence the administration of the RNAi agent, such as the amount of the RNAi agent, the timing of the administration of the RNAi agent, etc.

In certain embodiments, said RNAi agent is for use as described herein, wherein the methods may comprise evaluating the subject between one or more of the doses of the RNAi agent. The evaluation may comprise performing measurements on one or more biochemical parameters of the subject, including lipid parameters, between the doses, for example, one day, two days, three days, four days, five days, six days, seven days, eight day nine days, ten days, 11 days, 12 days, 13 days 14 days, 15 days, 20 days, 21 days, 28 days, 30 days, 35 days, 40 days, 42 days, 45 days, 49 days, 50 days, 56 days, 60 days, 63 days, 70 days, 77 days, 80 days, 84 days, and/or 90 days after administration of the RNAi agent. The biochemical measurements may include, but are not limited to, levels of LDL-C, HDL-C, PCSK9, total cholesterol, triglycerides, non-HDL-C, VLDL-C, Apo-A1, apolipoprotein B, Lp(a), CRP, glycated hemoglobin A1c, alanine aminotransferase, aspartate aminotransferase, alkaline aminotransferase, creatine kinase, and total bilirubin.

In some embodiments, the evaluation between one or more of the doses of the RNAi agent may measure physiological parameters or characteristics of the subject, including but not limited to weight, body mass index, heart rate, blood pressure, electrocardiogram parameters, etc.

In some embodiments, the results of the evaluation may determine and/or influence the subsequent administration or subsequent administrations of the RNAi agent, such as the amount of the RNAi agent, the timing of the administration of the RNAi agent, etc. For example, one or more results from the measurements may increase or decrease the subsequent dose of RNAi agent by about 5 %, or about 10 %, or about 20 %, or about 30 %, or about 40 %, or about 50 %, or about 60 %, or about 70 %, or about 80 %, or about 90 %, or greater.

Various biochemical parameters may determine/influence the dose of the RNAi agent to be administered. For instance, a measurement of alanine aminotransferase or aspartate aminotransferase that is over twice the ULN, and/or total bilirubin that is over 1.5 times the ULN, may result in lowering the dose of the subsequent administration of RNAi agent. As another example, a measurement of glycated hemoglobin A1c that is over 10 % may result in lowering the dose of the subsequent administration of RNAi agent. A measurement of LDL-C that is reduced by less than about 1 %, or less than about 5 %, or less than about 10 %, or less than about 15 %, from baseline measurements may result in increasing the dose of the subsequent administration of the RNAi agent. The dose may be lowered or increased, for instance, by about 1 %, or about 5 %, or about 10 %, or about 15 %, or about 20 %, or about 25 %, or about 30 %, or about 35 %, or about 40 %, or about 45 %, or about 50 %, or more.

In certain embodiments, after evaluating the subject before administration of the RNAi agent as described above, the subject may be administered a lipid-lowering therapy. The lipid-lowering therapy may be a treatment regimen, in which the subject is administered the lipid lowering therapy in regular intervals. In some embodiments, the subject may not have prior experience with a lipid-lowering therapy. In certain embodiments, after administration of the lipid-lowering treatment regimen, the subject may undergo an additional evaluation to perform measurements on one or more biochemical parameters of the subject, including lipid parameters, as described above. The subject may then be administered the RNAi agent as described herein.

### Administration of the RNAi Agent

The present invention provides an interfering ribonucleic acid (RNAi) agent for use in (i) the treatment of an atherosclerotic cardiovascular disease (ASCVD), an ASCVD risk equivalent, an elevated risk for cardiovascular disease (CVD), heterozygous familial hypercholesterolemia, or homozygous familial hypercholesterolemia, in a subject, or (ii) the prevention of a cardiovascular event, or the prevention of the development of an ASCVD, in a subject;
wherein the RNAi agent is a double-stranded ribonucleic acid comprising a sense strand and an antisense strand that forms a double-stranded region, the antisense strand comprising the nucleotide sequence of SEQ ID NO: 3, and the sense strand comprising the nucleotide sequence of SEQ ID NO: 4, wherein SEQ ID NO: 3 is 5'-asCfsaAfAfAfgCfaAfaAfcAfgGfuCfuagsasa-3', in which a, g, c, and u are 2'-O-methyl (2'-OMe) A, G, C, and U, respectively; Af, Gf, Cf, and Uf are 2'-fluoro A, G, C, and U, respectively; and s is a phosphorothioate linkage, and SEQ ID NO: 4 is 5'-csusagacCfuGfudTuugcuuuugu-3', in which a, g, c, and u are 2'-O-methyl (2'-OMe) A, G, C, and U, respectively; Af, Gf, Cf, and Uf are 2'-fluoro A, G, C, and U, respectively; dT is 2'-deoxythymidine; and s is a phosphorothioate linkage, and wherein the double-stranded ribonucleic acid has a covalently attached triantennary GalNAc ligand; and
wherein the RNAi agent is to be administered as a subcutaneous injection in a dosing regimen that comprises a loading phase followed by a maintenance phase, wherein the loading phase comprises administering the RNAi agent as at least two doses separated by a time interval, wherein the time interval is about 90 days.

The present invention comprises administering to subjects an effective amount, such as a prophylactically effective amount or a therapeutically effective amount, of said RNAi agent.

A "prophylactically effective amount" may include the amount of said RNAi agent that, when administered to a subject who does not yet experience or display symptoms of a condition, but who may be predisposed to the condition, is sufficient to prevent or ameliorate the condition or one or more symptoms of the condition. Ameliorating the condition includes slowing the course of the condition or reducing the severity of later-developing condition. The "prophylactically effective amount" may vary depending on the RNAi agent, how the agent is administered, the degree of risk of the condition, and the history, age, weight, family history, genetic makeup, the types of preceding or concomitant treatments, if any, and other individual characteristics of the patient to be treated.

A "therapeutically effective amount" may include the amount of said RNAi agent that, when administered to the subject, is sufficient to effect treatment of a condition (e.g., by diminishing, ameliorating or maintaining the existing condition or one or more symptoms of the condition). The "therapeutically effective amount" may vary depending on the RNAi agent, how the agent is administered, the condition and its severity and the history, age, weight, family history, genetic makeup, stage of pathological processes, the types of preceding or concomitant treatments, if any, and other individual characteristics of the patient to be treated.

"Baseline" may refer to a condition without treatment, such as before the treatment was administered.

The RNAi agent may be administered to a subject as a fixed dose. A "fixed dose" (e.g., a dose in mg) is a dose that is used for all subjects regardless of any specific subject-related factors, such as weight. Alternatively, the RNAi agent may be administered to a subject as a weight-based dose (e.g., a dose in mg/kg), which is a dose of the RNAi agent that will change depending on the subject's weight. In embodiments in which a subject receives multiple doses, the RNAi agent may be administered a combination of fixed doses and weight-based doses.

In certain embodiments, the RNAi agent is administered to the subject as a fixed dose of about 50 mg to about 800 mg, about 100 mg to about 800 mg, about 150 mg to about 800 mg, about 200 mg to about 800 mg, about 250 mg to about 800 mg, about 300 mg to about 800 mg, about 350 mg to about 800 mg, about 400 mg to about 800 mg, about 450 mg to about 800 mg, about 500 mg to about 800 mg, about 550 mg to about 800 mg, about 600 mg to about 800 mg, about 650 mg to about 800 mg, about 700 mg to about 800 mg, about 750 mg to about 800 mg, about 50 mg to about 750 mg, about 100 mg to about 750 mg, about 150 mg to about 750 mg, about 200 mg to about 750 mg, about 250 mg to about 750 mg, about 300 mg to about 750 mg, about 350 mg to about 750 mg, about 400 mg to about 750 mg, about 450 mg to about 750 mg, about 500 mg to about 750 mg, about 550 mg to about 750 mg, about 600 mg to about 750 mg, about 650 mg to about 750 mg, about 700 mg to about 750 mg, about 50 mg to about 700 mg, about 100 mg to about 700 mg, about 150 mg to about 700 mg, about 200 mg to about 700 mg, about 250 mg to about 700 mg, about 300 mg to about 700 mg, about 350 mg to about 700 mg, about 400 mg to about 700 mg, about 450 mg to about 700 mg, about 500 mg to about 700 mg, about 550 mg to about 700 mg, about 600 mg to about 700 mg, about 650 mg to about 700 mg, about 50 mg to about 650 mg, about 100 mg to about 650 mg, about 150 mg to about 650 mg, about 200 mg to about 650 mg, about 250 mg to about 650 mg, about 300 mg to about 650 mg, about 350 mg to about 650 mg, about 400 mg to about 650 mg, about 450 mg to about 650 mg, about 500 mg to about 650 mg, about 550 mg to about 650 mg, about 600 mg to about 650 mg, about 50 mg to about 600 mg, about 100 mg to about 600 mg, about 150 mg to about 600 mg, about 200 mg to about 600 mg, about 250 mg to about 600 mg, about 300 mg to about 600 mg, about 350 mg to about 600 mg, about 400 mg to about 600 mg, about 450 mg to about 600 mg, about 500 mg to about 600 mg, about 550 mg to about 600 mg, about 50 mg to about 550 mg, about 100 mg to about 550 mg, about 150 mg to about 550 mg, about 200 mg to about 550 mg, about 250 mg to about 550 mg, about 300 mg to about 550 mg, about 350 mg to about 550 mg, about 400 mg to about 550 mg, about 450 mg to about 550 mg, about 500 mg to about 550 mg, about 50 mg to about 500 mg, about 100 mg to about 500 mg, about 150 mg to about 500 mg, about 200 mg to about 500 mg, about 250 mg to about 500 mg, about 300 mg to about 500 mg, about 350 mg to about 500 mg, about 400 mg to about 500 mg, about 450 mg to about 500 mg, about 50 mg to about 450 mg, about 100 mg to about 450 mg, about 150 mg to about 450 mg, about 200 mg to about 450 mg, about 250 mg to about 450 mg, about 300 mg to about 450 mg, about 350 mg to about 450 mg, about 400 mg to about 450 mg, about 50 mg to about 400 mg, about 100 mg to about 400 mg, about 150 mg to about 400 mg, about 200 mg to about 400 mg, about 250 mg to about 400 mg, about 300 mg to about 400 mg, about 350 mg to about 400 mg, about 50 mg to about 350 mg, about 100 mg to about 350 mg, about 150 mg to about 350 mg, about 200 mg to about 350 mg, about 250 mg to about 350 mg, about 300 mg to about 350 mg, about 50 mg to about 300 mg, about 100 mg to about 300 mg, about 150 mg to about 300 mg, about 200 mg to about 300 mg, or about 250 mg to about 300 mg, e.g., a fixed dose of about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, or about 800 mg. Values and ranges intermediate to the foregoing recited values are also intended to be part of this invention.

The RNAi agent is administered as multiple doses as defined in the appended claims.

In the present invention, the RNAi agent is administered in a dosing regimen that includes a "loading phase" of closely spaced administrations as defined in the appended claims, that is followed by a "maintenance phase". In the "maintenance phase", the RNAi agent may be administered at longer spaced intervals.

In the present invention, the loading phase comprises administering the RNAi agent as at least two doses separated by a time interval, wherein the time interval is about 90 days. The loading phase comprises a first administration of the RNAi agent at Day 1, and then one or more administrations of the RNAi agent after about 90 days. The loading phase comprises administration of the RNAi agent at Day 1, and then a second administration of the RNAi agent after about three months. In some embodiments of the present invention, the loading phase may comprise additional doses of the RNAi agent (i.e., greater than two doses), and the interval between each administration may vary.

In the present invention, the RNA agent is administered at Day 1 and again at about Day 90.

In certain embodiments of the present invention, the RNAi agent may be administered to the subject during a loading phase as a fixed dose of about 50 mg to about 800 mg, about 100 mg to about 800 mg, about 150 mg to about 800 mg, about 200 mg to about 800 mg, about 250 mg to about 800 mg, about 300 mg to about 800 mg, about 350 mg to about 800 mg, about 400 mg to about 800 mg, about 450 mg to about 800 mg, about 500 mg to about 800 mg, about 550 mg to about 800 mg, about 600 mg to about 800 mg, about 650 mg to about 800 mg, about 700 mg to about 800 mg, about 750 mg to about 800 mg, about 50 mg to about 750 mg, about 100 mg to about 750 mg, about 150 mg to about 750 mg, about 200 mg to about 750 mg, about 250 mg to about 750 mg, about 300 mg to about 750 mg, about 350 mg to about 750 mg, about 400 mg to about 750 mg, about 450 mg to about 750 mg, about 500 mg to about 750 mg, about 550 mg to about 750 mg, about 600 mg to about 750 mg, about 650 mg to about 750 mg, about 700 mg to about 750 mg, about 50 mg to about 700 mg, about 100 mg to about 700 mg, about 150 mg to about 700 mg, about 200 mg to about 700 mg, about 250 mg to about 700 mg, about 300 mg to about 700 mg, about 350 mg to about 700 mg, about 400 mg to about 700 mg, about 450 mg to about 700 mg, about 500 mg to about 700 mg, about 550 mg to about 700 mg, about 600 mg to about 700 mg, about 650 mg to about 700 mg, about 50 mg to about 650 mg, about 100 mg to about 650 mg, about 150 mg to about 650 mg, about 200 mg to about 650 mg, about 250 mg to about 650 mg, about 300 mg to about 650 mg, about 350 mg to about 650 mg, about 400 mg to about 650 mg, about 450 mg to about 650 mg, about 500 mg to about 650 mg, about 550 mg to about 650 mg, about 600 mg to about 650 mg, about 50 mg to about 600 mg, about 100 mg to about 600 mg, about 150 mg to about 600 mg, about 200 mg to about 600 mg, about 250 mg to about 600 mg, about 300 mg to about 600 mg, about 350 mg to about 600 mg, about 400 mg to about 600 mg, about 450 mg to about 600 mg, about 500 mg to about 600 mg, about 550 mg to about 600 mg, about 50 mg to about 550 mg, about 100 mg to about 550 mg, about 150 mg to about 550 mg, about 200 mg to about 550 mg, about 250 mg to about 550 mg, about 300 mg to about 550 mg, about 350 mg to about 550 mg, about 400 mg to about 550 mg, about 450 mg to about 550 mg, about 500 mg to about 550 mg, about 50 mg to about 500 mg, about 100 mg to about 500 mg, about 150 mg to about 500 mg, about 200 mg to about 500 mg, about 250 mg to about 500 mg, about 300 mg to about 500 mg, about 350 mg to about 500 mg, about 400 mg to about 500 mg, about 450 mg to about 500 mg, about 50 mg to about 450 mg, about 100 mg to about 450 mg, about 150 mg to about 450 mg, about 200 mg to about 450 mg, about 250 mg to about 450 mg, about 300 mg to about 450 mg, about 350 mg to about 450 mg, about 400 mg to about 450 mg, about 50 mg to about 400 mg, about 100 mg to about 400 mg, about 150 mg to about 400 mg, about 200 mg to about 400 mg, about 250 mg to about 400 mg, about 300 mg to about 400 mg, about 350 mg to about 400 mg, about 50 mg to about 350 mg, about 100 mg to about 350 mg, about 150 mg to about 350 mg, about 200 mg to about 350 mg, about 250 mg to about 350 mg, about 300 mg to about 350 mg, about 50 mg to about 300 mg, about 100 mg to about 300 mg, about 150 mg to about 300 mg, about 200 mg to about 300 mg, or about 250 mg to about 300 mg, e.g., a fixed dose of about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, or about 800 mg. Values and ranges intermediate to the foregoing recited values are also intended to be part of this invention.

In some embodiments of the present invention, each dose of the RNAi agent administered during the loading phase is the same dosage amount, or it may differ.

In certain embodiments of the present invention, the RNAi agent may be administered as a dose of about 300 mg on Day 1 and as a dose of about 300 mg on about Day 90, or on Day 90.

In certain embodiments of the present invention, the maintenance phase may comprise administration of one or more doses of the RNAi agent to the subject. The administration may be once a month, once every two months, once every three months, once every four months, once every five months, once every six months, once every seven months, once every eight months, once every nine months, once every ten months, once every 11 months, once every 12 months, once every year, once every 13 months, once every 14 months, once every 15 months, once every 16 months, once every 17 months, once every 18 months, once every 19 months, once every 20 months, once every 21 months, once every 22 months, once every 23 months, once every 24 months, once every 25 months, once every 26 months, once every 27 months, once every 28 months, once every 29 months, once every 30 months, once every 31 months, once every 32 months, once every 33 months, once every 34 months, once every 35 months, once every 36 months, once every 37 months, once every 38 months, once every 39 months, once every 40 months, once every 41 months, once every 42 months, once every 43 months, once every 44 months, once every 45 months, once every 46 months, once every 47 months, once every 48 months, or longer. In one particular embodiment, the maintenance dose is administered to the subject once every three months, or once every six months, or once every nine months, or once every year.

In embodiments of the invention, the maintenance phase may comprise administration of the RNAi agent as multiple doses that repeat, for example, at regular intervals. For instance, the RNAi agent may be administered to the subject at an interval of about one day, about two days, about three days, about four days, about five days, about six days, about a week, about two weeks, about three weeks, about four weeks, about one month, about two months, about three months, about four months, about five months, about six months, about seven months, about eight months, about nine months, about ten months, about 11 months, about 12 months, about one year, about 13 months, about 14 months, about 15 months, about 16 months, about 17 months, about 18 months, about 19 months, about 20 months, about 21 months, about 22 months, about 23 months, about 24 months, or longer, e.g., chronic administration. In certain embodiments, the fixed dose may be administered to the subject one or more times per year, i.e., twice, three times, four times, five times, six times, seven times, eight times, nine times, 10 times, 11 times, 12 times, 13 times, 14 times, 15 times, 16 times, 17 times, 18 times, 19 times, 20 times, 21 times, 22 times, 23 times, 24 times, or more often. In some embodiments, the fixed dose may be administered to the subject once every about four weeks, every about five weeks, every about six weeks, every about seven weeks, every about eight weeks, every about nine weeks, every about 10 weeks, every about 11 weeks, every about 12 weeks, every about 13 weeks, every about 14 weeks, every about 15 weeks, every about 16 weeks, every about 17 weeks, every about 18 weeks, every about 19 weeks, every about 20 weeks, every about 22 weeks, every about 24 weeks, every about 26 weeks, every about 28 weeks, every about 30 weeks, every about 32 weeks, every about 34 weeks, every about 36 weeks, every about 38 weeks, every about 40 weeks, every about 42 weeks, every about 44 weeks, every about 46 weeks, every about 48 weeks, every about 50 weeks, every about 52 weeks, or longer. In certain embodiments, the fixed dose may be administered to the subject once per day, once per about two days, once per about three days, once per about four days, once per about five days, once per about six days, once per about seven days, once per about eight days, once per about nine days, once per about 10 days, once per about 11 days, once per about 12 days, once per about 13 days, once per about 14 days, once per about 15 days, once per about 16 days, once per about 17 days, once per about 18 days, once per about 19 days, once per about 20 days, once per about 30 days, once per about 40 days, once per about 50 days, once per about 60 days, once per about 70 days, once per about 80 days, once per about 90 days, once per about 100 days, once per about 110 days, once per about 120 days, once per about 130 days, once per about 140 days, once per about 150 days, once per about 160 days, once per about 170 days, once per about 180 days, once per about 190 days, once per about 200 days, once per about 210 days, once per about 220 days, once per about 230 days, once per about 240 days, once per about 250 days, once per about 260 days, once per about 270 days, once per about 280 days, once per about 290 days, once per about 300 days, once per about 310 days, once per about 320 days, once per about 330 days, once per about 340 days, once per about 350 days, once per about 360 days, once per about 365 days, or longer.

In certain embodiments, the maintenance phase may comprise administration of a dose of the RNAi agent to the subject every about three months, every about four months, every about six months, every about nine months, or every about year.

The maintenance dose or doses may be the same or different from the loading dose or doses. For instance, a maintenance dose may be about 25 mg to about 800 mg administered to the subject, for example about 25 mg, about 50 mg, about 75 mg, about 100 mg, about 125 mg, about 150 mg, about 175 mg, about 200 mg, 225 mg, about 250 mg, about 275 mg, about 300 mg, 325 mg, about 350 mg, about 375 mg, about 400 mg, 425 mg, about 450 mg, about 475 mg, about 500 mg, 525 mg, about 550 mg, about 575 mg, about 600 mg, 625 mg, about 650 mg, about 675 mg, about 700 mg, 725 mg, about 750 mg, about 775 mg, or about 800 mg. Values and ranges intermediate to the foregoing recited values are also intended to be part of this invention.

In certain embodiments, the maintenance phase may comprise administration of a 300 mg dose of the RNAi agent to the subject every three months, every four months, every six months, every nine months, or every year.

In certain embodiment of the present invention, the RNAi agent may be administered in a dosing regimen that comprises (1) a loading phase, in which the RNAi agent is administered as a 300 mg dose on Day 1 and on about Day 90; and (2) a maintenance phase, in which the RNAi agent is administered as a 300 mg every about six months following the administration on about Day 90.

The RNAi agent is administered subcutaneously.

In some embodiments, the administration is via a subcutaneous depot injection..

In some embodiments, the administration is via a pump. The pump may be an external pump or a surgically implanted pump. In certain embodiments, the pump is a subcutaneously implanted osmotic pump. In other embodiments, the pump is a subcutaneous infusion pump.

The RNAi agent may be administered in combination with one or more other prophylactic or therapeutic agents. The RNAi agent may be in the same formulation as the one or more other prophylactic or therapeutic agents, or the RNAi agent may be in a different formulation than the one or more other prophylactic or therapeutic agents. The RNAi agent and the one or more other prophylactic or therapeutic agents may be administered concurrently, or within a short time period of each other, e.g., within about 1 minute, or about 5 minutes, or about 15 minutes, or about 30 minutes, or about 60 minutes, or about two hours, or about three hours, or about four hours, or about six hours, or about nine hours, or about 12 hours, or about 15 hours, or about 18 hours, or about 24 hours.

The one or more other prophylactic or therapeutic agents include those known to treat lipid disorders, such as hypercholesterolemia, atherosclerosis, or dyslipidemia. For example, the one or more other prophylactic or therapeutic agents may be an HMG-CoA reductase inhibitor, a fibrate, a bile acid sequestrant, niacin, an antiplatelet agent, an angiotensin converting enzyme inhibitor, an angiotensin II receptor antagonist, an acylCoA cholesterol acetyltransferase (ACAT) inhibitor, a cholesterol absorption inhibitor, a cholesterol ester transfer protein (CETP) inhibitor, a microsomal triglyceride transfer protein (MTTP) inhibitor, a cholesterol modulator, a bile acid modulator, a peroxisome proliferation activated receptor (PPAR) agonist, a gene-based therapy, a composite vascular protectant (e.g., AGI-1067, from Atherogenics), a glycoprotein Ilb/IIIa inhibitor, aspirin or an aspirin- like compound, an IBAT inhibitor, a squalene synthase inhibitor, or a monocyte chemoattractant protein (MCP)-I inhibitor. As a further example, the one or more other prophylactic or therapeutic agents may be an anti-PCSK9 antibody, such as alirocumab (Praluent), evolocumab (Repatha), bococizumab, lodelcizumab, ralpancizumab, RG7652, LY3015014, LPD1462, AX1, ALD306, or Ig1-PA4.

### Subject Administered the RNAi Agent

The subject administered the RNAi agent may be a human or non-human animal, in certain embodiments a vertebrate, and in particular embodiments a mammal. In preferred embodiments, the subject is a human. In certain embodiments, the subject is an adult. In some embodiments, the subject is a patient.

The subject may have a baseline LDL-C level of about 70 mg/dl or greater. In certain embodiments, the baseline LDL-C level is about 80 mg/dl or greater, or about 90 mg/dl or greater, or about 100 mg/dl or greater, or about 110 mg/dl or greater, or about 120 mg/dl or greater, or about 130 mg/dl or greater, or about 140 mg/dl or greater, or about 150 mg/dl or greater, or about 160 mg/dl or greater, or about 170 mg/dl or greater, or about 180 mg/dl or greater, or about 190 mg/dl or greater, or about 200 mg/dl or greater, or about 210 mg/dl or greater, or about 220 mg/dl or greater, or about 230 mg/dl or greater, or about 240 mg/dl or greater, or about 250 mg/dl or greater, or about 260 mg/dl or greater, or about 270 mg/dl or greater, or about 280 mg/dl or greater, or about 290 mg/dl or greater, or about 300 mg/dl or greater.

In some embodiments, the subject requires lowering of LDL-C. Subjects who may require lowering of LDL-C may have an LDL-C level of about 50 mg/dl or greater, or about 60 mg/dl or greater, or about 70 mg/dl or greater, or about 80 mg/dl or greater, or about 90 mg/dl or greater, or about 100 mg/dl or greater, or about 110 mg/dl or greater, or about 120 mg/dl or greater, or about 130 mg/dl or greater, or about 140 mg/dl or greater, or about 150 mg/dl or greater, or about 160 mg/dl or greater, or about 170 mg/dl or greater, or about 180 mg/dl or greater, or about 190 mg/dl or greater, or about 200 mg/dl or greater, or about 210 mg/dl or greater, or about 220 mg/dl or greater, or about 230 mg/dl or greater, or about 240 mg/dl or greater, or about 250 mg/dl or greater, or about 260 mg/dl or greater, or about 270 mg/dl or greater, or about 280 mg/dl or greater, or about 290 mg/dl or greater, or about 300 mg/dl or greater.

In some embodiments, the subject does not have active liver disease. Active liver disease may be determined by measuring one or more biochemical parameters of alanine aminotransferase, aspartate aminotransferase, and total bilirubin. The biochemical parameters may be measured at baseline. In certain embodiments, the subject may have an alanine aminotransferase level of no greater than twice (2x) the ULN. In some embodiments, the subject may have an alanine aminotransferase level of about 1.5x the ULN, or about the same as the ULN, or less than the ULN.

In some embodiments, the subject may have an aspartate aminotransferase level of no greater than twice (2x) the ULN. In certain embodiments, the subject may have an aspartate aminotransferase level of about 1.5x the ULN, or about the same as the ULN, or less than the ULN.

In some embodiments, the subject may have a total bilirubin level of no greater than 1.5x the ULN. In certain embodiments, the subject may have a total bilirubin level of about the same as the ULN, or less than the ULN.

Alternatively, the subject may have active liver disease. In certain embodiments, the subject may have an alanine aminotransferase level of greater than twice (2x) the ULN, such as about 2.5x the ULN, or about 3x the ULN, or about 3.5x the ULN, or about 4x the ULN, or greater. In some embodiments, the subject may have an aspartate aminotransferase level of greater than twice (2x) the ULN, such as about 2.5x the ULN, or about 3x the ULN, or about 3.5x the ULN, or about 4x the ULN, or greater. In certain embodiments, the subject may have a total bilirubin level of greater than 1.5x the ULN, such as about 2x the ULN, or about 2.5x the ULN, or about 3x the ULN, or about 3.5x the ULN, or about 4x the ULN, or greater.

The ULN of alanine aminotransferase, aspartate aminotransferase, and total bilirubin would be understood by a person of ordinary skill in the art.

In some embodiments, the subject may be on a background lipid-lowering therapy. The subject may continue receiving the background lipid-lowering therapy while being administered the RNAi agent, or the subject may cease the background lipid-lowering therapy. In certain embodiments, the background lipid-lowering therapy may be a statin, examples of which include, but are not limited to atorvastatin, pravastatin, simvastatin, lovastatin, fluvastatin, cerivastatin, rosuvastatin, and pitivastatin. The subject may be on maximally tolerated statin therapy; the maximum dosages for statins are known in the art.

In some embodiments, the subject may be on another type of lipid-lowering therapy, such as ezetimibe, LDL apheresis, bile acid sequestrants, nicotinic acid, and fibrates.

In other embodiments, the subject is not on a background lipid-lowering therapy.

In certain embodiments, the subject may be on a diet, for example, a diet designed to improve lipid levels. Such diets are known in the art. For instance, a diet designed to improve lipid levels may comprise eating lean cuts of meat; removing fat from meats; not eating fried foods or high-fat sauces; not eating egg yolks; using low-fat dairy products such as skim milk or 1% milk, low-fat frozen yogurt, low-fat ice cream and low-fat cheeses; and eating foods that are sources of fiber, such as fruits and vegetables.

The subject may have a triglyceride level of no greater than about 400 mg/dl. For example, the subject may have a baseline triglyceride level of about 380 mg/dl, or about 360 mg/dl, or about 340 mg/dl, or about 320 mg/dl, or about 300 mg/dl, or about 280 mg/dl, or about 260 mg/dl, or about 240 mg/dl, or about 220 mg/dl, or about 200 mg/dl, or about 180 mg/dl, or about 160 mg/dl, or about 140 mg/dl, or about 120 mg/dl, or about 100 mg/dl, or about 80 mg/dl, or about 60 mg/dl.

In other embodiments, the subject may have a triglyceride level of greater than about 400 mg/dl. For instance, the subject may have a baseline triglyceride level of about 420 mg/dl, or about 440 mg/dl, or about 460 mg/dl, or about 480 mg/dl, or about 500 mg/dl, or about 520 mg/dl, or about 540 mg/dl, or about 560 mg/dl, or about 580 mg/dl, or about 600 mg/dl.

In some embodiments, the triglyceride level is measured at baseline.

The subject may have an estimated glomerular filtration rate (eGFR) of at least about 30 ml/min. For example, the eGFR may be about 30 ml/min, or about 35 ml/min, or about 40 ml/min, or about 45 ml/min, or about 50 ml/min, or greater.

In some embodiments, the subject has hyperlipidemia, such as hypercholesterolemia. In certain embodiments, the subject has heterozygous familial hypercholesterolemia. In other embodiment, the subject has homozygous familial hypercholesterolemia.

Alternatively, the subject may have an eGFR of less than about 30 ml/min, such as an eGFR of about 25 ml/min, or about 20 ml/min, or about 15 ml/min.

In some embodiments, eGFR is measured at baseline.

The subject may not have Type 2 diabetes that is poorly controlled, such as Type 2 diabetes that is not being treated or addressed properly, or at all. Poorly controlled diabetes may be identified by a glycated hemoglobin A1c level of at least about 10 %. Therefore, the subject may have a glycated hemoglobin A1c level of less than about 10 %, such as about 9 %, or about 8 %, or about 7 %, or about 6 %, or about 5 %, or less.

In other embodiments, the subject may have Type 2 diabetes that is poorly controlled, which can be evidenced by a glycated hemoglobin A1c level of at least about 10 %, such as about 15 %, or about 20 %, or about 25 %, or about 30 % or greater.

In some embodiments, the glycated hemoglobin A1c level is measured at baseline.

The subject may not have heart failure that is characterized by the New York Heart Association (NYHA) as class II, III, or IV. Alternatively, the subject may be suffering from heart failure that is characterized by the New York Heart Association (NYHA) as class II, III, or IV.

The subject may have ventricular ejection fraction of 30 % or greater, such as about 35 % or about 40 %, or about 45 %, or about 50 %, or greater. In other embodiments, the subject may have ventricular ejection fraction of less than 30 %, such as a ventricular ejection fraction of about 25 %, or about 20 %, or about 15 %, or less. The ventricular ejection fraction may be measured as a baseline measurement, or it may be the last known ventricular ejection fraction that was measured previously.

The subject may not have experienced a major adverse cardiac event within six months of administration of the RNAi agent. Major adverse cardiac events include, but are not limited to, death, nonfatal myocardial infarction, severe recurrent ischemia, stroke, symptomatic pulmonary embolism, and bleeding. In some embodiments, the major adverse cardiac event did not occur within about seven months, about eight months, about nine months, about ten months, about 11 months, about 12 months, about 18 months, about 24 months, about 30 months, about 36 months, or greater, of the administration of the RNAi agent, including no occurrence ever of a major cardiac event. In alternative embodiments, the subject may have experienced a major cardiac event within six months of administration of the RNAi event, including within about 5 months, or about 4 months, or about 3 months, or about 2 months, or about 1 month, or about 4 weeks, or about 3 weeks, or about 2 weeks, or about 1 week, or sooner.

The subject may not have severe hypertension. In some embodiments, severe hypertension may be identified by a systolic blood pressure of greater than about 180 mmHg, such as systolic blood pressure of about 190 mmHg, or about 200 mmHg, or about 220 mmHg, or about 240 mmHg, or about 260 mmHg, or about 280 mmHg, or about 300 mmHg, or higher; and/or a diastolic blood pressure of greater than about 110 mmHg, such as diastolic blood pressure of about 120 mmHg, or about 140 mmHg, or about 160 mmHg, or about 180 mmHg, or about 200 mmHg, or about 220 mmHg, or about 240 mmHg, or higher. In certain embodiments, the subject may not have uncontrolled severe hypertension. Uncontrolled severe hypertension may be identified by a systolic blood pressure of greater than about 180 mmHg and/or a diastolic blood pressure of greater than about 110 mmHg despite anti-hypertensive therapy. Alternatively, the subject may have severe hypertension and/or uncontrolled severe hypertension.

In some embodiments, blood pressure is measured at baseline.

The subject may not have any history of a hemorrhagic stroke. Alternatively, the subject may have experienced a hemorrhagic stroke.

The subject may not have had a cardiac arrhythmia within three months of administration of the RNAi agent, including within about four months, or about five months, or about six months, or about seven months, or about eight months, or about nine months, or about ten months, or about 11 months, or about 12 months, or longer. In other embodiments, the subject may have had a cardiac arrhythmia within three months of administration of the RNAi agent, but the cardiac arrhythmia was controlled, for example, by medication or via ablation. Alternatively, the subject may have had a cardiac arrhythmia within three months that was not controlled, for instance, by medication or via ablation.

In certain embodiments, the subject may have one or more of the following: a history of myocardial infarction, stable or unstable angina, coronary or other arterial revascularization, stroke, transient ischemic attack, or peripheral arterial disease of atherosclerotic origin; being male; having a family history of heart disease, ASCVD, or ASCVD risk equivalent; having a smoking habit; being physically inactive; having high blood pressure; having high blood cholesterol; having diabetes and prediabetes; being overweight or obese; having a history of preeclampsia during pregnancy; having uncontrolled stress and/or anger; being post-menopausal; having an unhealthy diet, e.g., a diet high in salt, saturated fat, trans fat, cholesterol, and/or refined sugars; being age 55 or older; having sleep apnea; having anemia; or a combination thereof.

In some embodiments, the subject may have cognitive impairment, such as Alzheimer's disease, dementia, memory loss, etc. In other embodiments, the subject does not have any cognitive impairment.

### RNAi Agent

The RNAi agent is a double-stranded ribonucleic acid comprising a sense strand and an antisense strand that forms a double-stranded region.

The double-stranded ribonucleic acid comprises a ligand. In certain embodiments, the ligand is conjugated to the 3' end of the sense strand of the double-stranded ribonucleic acid. Ligands are described in PCT Application No. PCT/US2016/048666 filed on August 25, 2016, and in U.S. Application Serial No. 14/650,128 filed June 5, 2015. The ligand is an N-acetylgalactosamine (GalNAc) derivative.

The RNAi agent is a double-stranded ribonucleic acid comprising an antisense strand of the nucleotide sequence of 5'- asCfsaAfAfAfgCfaAfaAfcAfgGfuCfuagsasa - 3' (SEQ ID NO: 3) and a sense strand of the nucleotide sequence of 5' - csusagacCfuGfudTuugcuuuugu - 3' (SEQ ID NO: 4), in which a, g, c and u are 2'-O-methyl (2'-OMe) A, G, C, or U; Af, Gf, Cf or Uf are 2'-fluoro A, G, C or U; dT is 2'-deoxythymidine; and s is a phosphorothioate linkage. The double-stranded ribonucleic acid has a covalently attached triantennary GalNAc ligand.

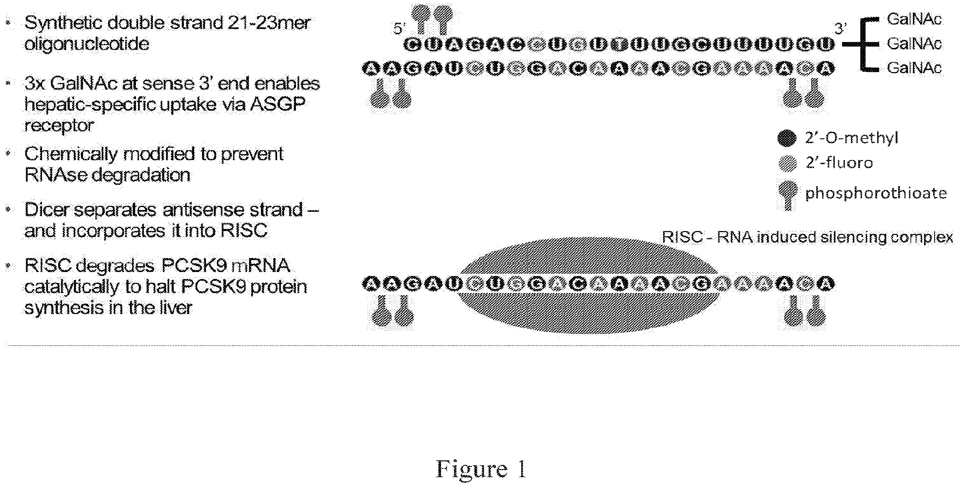

In some embodiments, the RNAi agent is as depicted in Figure 1.

The RNAi agent may be in "naked" form, or as a "free RNA." The "naked" form refers to the absence of a pharmaceutical composition. For instance, the naked RNAi agent may be in a suitable buffer solution, which may comprise, as examples, acetate, citrate, prolamine, carbonate, or phosphate, or any combination thereof. In some embodiments, the buffer solution is phosphate buffered saline (PBS). The pH and osmolarity of the buffer solution containing the RNAi agent can be adjusted such that it is suitable for administering to a subject. In certain embodiments, the RNAi agent may be in water for injection.

Alternatively, the RNAi agent may be formulated in a pharmaceutical composition that comprises the RNAi agent and one or more pharmaceutically acceptable carriers. The pharmaceutical compositions may be formulated based on the mode of delivery. For example, the compositions may be formulated for systemic administration via parenteral delivery, by subcutaneous injection or infusion.

Compositions and formulations for parenteral administration may include, but are not limited to, sterile aqueous solutions which can also contain buffers, diluents and other suitable additives such as penetration enhancers, carrier compounds and other pharmaceutically acceptable carriers or excipients.

Examples of pharmaceutically acceptable carriers and particular compositions are described in PCT Application No. PCT/US2016/048666 filed on August 25, 2016, and in U.S. Application Serial No. 14/650,128 filed June 5, 2015.

In certain embodiments, the RNAi agent may be formulated in a suitable concentration such that administration delivers a fixed dose of about 50 mg to about 800 mg, about 100 mg to about 800 mg, about 150 mg to about 800 mg, about 200 mg to about 800 mg, about 250 mg to about 800 mg, about 300 mg to about 800 mg, about 350 mg to about 800 mg, about 400 mg to about 800 mg, about 450 mg to about 800 mg, about 500 mg to about 800 mg, about 550 mg to about 800 mg, about 600 mg to about 800 mg, about 650 mg to about 800 mg, about 700 mg to about 800 mg, about 750 mg to about 800 mg, about 50 mg to about 750 mg, about 100 mg to about 750 mg, about 150 mg to about 750 mg, about 200 mg to about 750 mg, about 250 mg to about 750 mg, about 300 mg to about 750 mg, about 350 mg to about 750 mg, about 400 mg to about 750 mg, about 450 mg to about 750 mg, about 500 mg to about 750 mg, about 550 mg to about 750 mg, about 600 mg to about 750 mg, about 650 mg to about 750 mg, about 700 mg to about 750 mg, about 50 mg to about 700 mg, about 100 mg to about 700 mg, about 150 mg to about 700 mg, about 200 mg to about 700 mg, about 250 mg to about 700 mg, about 300 mg to about 700 mg, about 350 mg to about 700 mg, about 400 mg to about 700 mg, about 450 mg to about 700 mg, about 500 mg to about 700 mg, about 550 mg to about 700 mg, about 600 mg to about 700 mg, about 650 mg to about 700 mg, about 50 mg to about 650 mg, about 100 mg to about 650 mg, about 150 mg to about 650 mg, about 200 mg to about 650 mg, about 250 mg to about 650 mg, about 300 mg to about 650 mg, about 350 mg to about 650 mg, about 400 mg to about 650 mg, about 450 mg to about 650 mg, about 500 mg to about 650 mg, about 550 mg to about 650 mg, about 600 mg to about 650 mg, about 50 mg to about 600 mg, about 100 mg to about 600 mg, about 150 mg to about 600 mg, about 200 mg to about 600 mg, about 250 mg to about 600 mg, about 300 mg to about 600 mg, about 350 mg to about 600 mg, about 400 mg to about 600 mg, about 450 mg to about 600 mg, about 500 mg to about 600 mg, about 550 mg to about 600 mg, about 50 mg to about 550 mg, about 100 mg to about 550 mg, about 150 mg to about 550 mg, about 200 mg to about 550 mg, about 250 mg to about 550 mg, about 300 mg to about 550 mg, about 350 mg to about 550 mg, about 400 mg to about 550 mg, about 450 mg to about 550 mg, about 500 mg to about 550 mg, about 50 mg to about 500 mg, about 100 mg to about 500 mg, about 150 mg to about 500 mg, about 200 mg to about 500 mg, about 250 mg to about 500 mg, about 300 mg to about 500 mg, about 350 mg to about 500 mg, about 400 mg to about 500 mg, about 450 mg to about 500 mg, about 50 mg to about 450 mg, about 100 mg to about 450 mg, about 150 mg to about 450 mg, about 200 mg to about 450 mg, about 250 mg to about 450 mg, about 300 mg to about 450 mg, about 350 mg to about 450 mg, about 400 mg to about 450 mg, about 50 mg to about 400 mg, about 100 mg to about 400 mg, about 150 mg to about 400 mg, about 200 mg to about 400 mg, about 250 mg to about 400 mg, about 300 mg to about 400 mg, about 350 mg to about 400 mg, about 50 mg to about 350 mg, about 100 mg to about 350 mg, about 150 mg to about 350 mg, about 200 mg to about 350 mg, about 250 mg to about 350 mg, about 300 mg to about 350 mg, about 50 mg to about 300 mg, about 100 mg to about 300 mg, about 150 mg to about 300 mg, about 200 mg to about 300 mg, or about 250 mg to about 300 mg, e.g., a fixed dose of about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, or about 800 mg. Values and ranges intermediate to the foregoing recited values are also intended to be part of this invention.

In some embodiments, the RNAi agent may be formulated in a suitable concentration such that a suitable volume of the composition is administered to the subject, such as about 1.0 ml, about 1.1 ml, about 1.2 ml, about 1.3 ml, about 1.4 ml, about 1.5 ml, about 1.6 ml, about 1.7 ml, about 1.8 ml, about 1.9 ml, or about 2.0 ml of a pharmaceutical composition. For example, in one embodiment, an RNAi agent is formulated in a suitable pharmaceutical formulation at about 200 mg/ml such that administration of about 1.5 ml of the formulation to a subject provides a 300 mg fixed dose of the agent.

Unless otherwise defined, all technical and scientific terms used herein have the same meaning as commonly understood by one of ordinary skill in the art to which this invention belongs.

### EXAMPLES

A placebo-controlled, double-blind, randomized trial to compare the effect of different doses of an RNAi agent given as single or multiple subcutaneous injections in patients with high cardiovascular risk and elevated LDL-C was conducted.

### Methods

The RNAi agent was a double-stranded ribonucleic acid comprising an antisense strand of the nucleotide sequence of 5'- asCfsaAfAfAfgCfaAfaAfcAfgGfuCfuagsasa - 3' (SEQ ID NO: 3) and a sense strand of the nucleotide sequence of 5' - csusagacCfuGfudTuugcuuuugu - 3' (SEQ ID NO: 4), in which a, g, c and u are 2'-O-methyl (2'-OMe) A, G, C, or U; Af, Gf, Cf or Uf are 2'-fluoro A, G, C or U; dT is 2'-deoxythymidine; and s is a phosphorothioate linkage. The double-stranded ribonucleic acid had a covalently attached triantennary GalNAc ligand.

The primary objective was to evaluate the effect of the RNAi agent treatment on LDL-C levels at Day 180. The secondary objective was to evaluate the effect of the RNAi agent on: (i) LDL-C levels at Day 90; (ii) LDL-C levels at other time points; (iii) PCSK9 levels over time; (iv) other lipids, lipoproteins, apolipoproteins; (v) proportion of patients achieving pre-specified global lipid guidelines; (vi) individual responsiveness to different doses; (vii) duration of lipid-lowering effect of different doses; and (viii) safety and tolerability profile of the RNAi agent. The study also collected/evaluated the effect of the RNAi agent on: (a) cardiovascular events such as cardiovascular death, non-fatal myocardial infarction, resuscitated cardiac arrest, and non-fatal stroke (ischemic and hemorrhagic); and (b) anti-drug antibodies for the RNAi agent.

The study involved 501 patients with ASCVD or ASCVD-risk equivalents (e.g., diabetes and familial hypercholesterolemia) and elevated LDL-C. Patients were included if they met all of the following inclusion criteria prior to randomization:
(a) male or female patients ≥ 18 years of age;
(b) history of ASCVD or ASCVD-risk equivalents (symptomatic atherosclerosis, Type 2 diabetes, familial hypercholesterolemia, including patients whose 10-year risk of a cardiovascular event assessed by Framingham Risk Score* or equivalent has a target LDL-C of < 100 mg/dl);
(c) serum LDL-C ≥ 1.8 mmol/l (≥ 70 mg/dl) for ASCVD patients or ≥ 2.6 mmol/l (≥ 100 mg/dl) for ASCVD-risk equivalent patients at screening;
(d) fasting triglyceride < 4.52 mmol/l (< 400 mg/dl) at screening;
(e) calculated glomerular filtration rate > 30 ml/min by estimated glomerular filtration rate (eGFR) using standardized local clinical methodology;
(f) patients on statins should be receiving a maximally tolerated dose (investigator's discretion);
(g) patients on lipid-lower therapies (such as statin and/or ezetimibe) should be on a stable dose for ≥ 30 days before screening with no planned medication or dose change during study participation; and
(h) willing and able to give informed consent before initiation of any study-related procedures and willing to comply with all required study procedures.
*By Framingham Risk Score > 20 %

Patients were excluded from the study if any of the following exclusion criteria apply immediately prior to randomization:
(a) any uncontrolled or serious disease, or any medical or surgical condition, that may either interfere with participation in the clinical study, and/or put the patient at significant risk (according to investigator's [or delegate] judgment) if he/she participates in the clinical study;
(b) an underlying known disease, or surgical, physical, or medical condition that, in the opinion of the investigator (or delegate) might interfere with interpretation of the clinical study results;
(c) New York Heart Association (NYHA) class II, III or IV heart failure or last known left ventricular ejection fraction <30 %;
(d) cardiac arrhythmia within 3 months prior to randomization that is not controlled by medication or via ablation;
(e) any history of hemorrhagic stroke;
(f) major adverse cardiac event within 6 months prior to randomization;
(g) uncontrolled severe hypertension: systolic blood pressure > 180 mmHg or diastolic blood pressure > 110 mmHg prior to randomization despite anti-hypertensive therapy;
(h) poorly controlled Type 2 diabetes, i.e., glycated hemoglobin A1c (HbA1c) > 10.0 % prior to randomization;
(i) active liver disease defined as any known current infectious, neoplastic, or metabolic pathology of the liver or unexplained alanine aminotransferase, aspartate aminotransferase, elevation > 2x the ULN, or total bilirubin elevation > 1.5x ULN at screening confirmed by a repeat measurement at least 1 week apart;
(j) serious comorbid disease in which the life expectancy of the patient is shorter than the duration of the trial (e.g., acute systemic infection, cancer, or other serious illnesses); this includes all cancers with the exception of treated basal-cell carcinoma occurring > 5 years before screening;
(k) females who are pregnant or nursing, or who are of childbearing potential and unwilling to use at least two methods of contraception (oral contraceptives, barrier methods, approved contraceptive implant, long- term injectable contraception, intrauterine device or tubal litigation)**; women who are > 2 years postmenopausal defined as ≥ 1 year since last menstrual period AND if less than 55 years old with a negative pregnancy test within 24 hours of randomization or surgically sterile are exempt from this exclusion;
(l) males who are unwilling to use an acceptable method of birth control during the entire study period (i.e., condom with spermicide);
(m) known history of alcohol and/or drug abuse;
(n) treatment with other investigational medicinal products or devices within 30 days or five half-lives, whichever is longer;
(o) use of other investigational medicinal products or devices during the course of the study;
(p) any condition that according to the investigator could interfere with the conduct of the study, such as but not limited to:
   (i) inappropriate for this study, including patients who are unable to communicate or to cooperate with the investigator;
   (ii) unable to understand the protocol requirements, instructions and study-related restrictions, the nature, scope, and possible consequences of the study (including patients whose cooperation is doubtful due to drug abuse or alcohol dependency);
   (iii) unlikely to comply with the protocol requirements, instructions, and study-related restrictions (e.g., uncooperative attitude, inability to return for follow-up visits, and improbability of completing the study);
   (iv) have any medical or surgical condition, which in the opinion of the investigator would put the patient at increased risk from participating in the study;
   (v) involved with, or a relative of, someone directly involved in the conduct of the study;
   (vi) any known cognitive impairment (e.g., Alzheimer's disease); and
(q) previous or current treatment (within 90 days of screening) with monoclonal antibodies directed towards PCSK9.
**For the entire duration of the study

The patients were screened and randomized into six RNAi agent groups and placebo groups. Treatment allocation was stratified by country and by current use of statins or other lipid-modifying therapies. Each patient received (i) one or two injections on Day 1 only of the RNAi agent or a placebo, or (ii) a single injection on Day 1 and a second injection on Day 90 of the RNAi agent or a placebo.

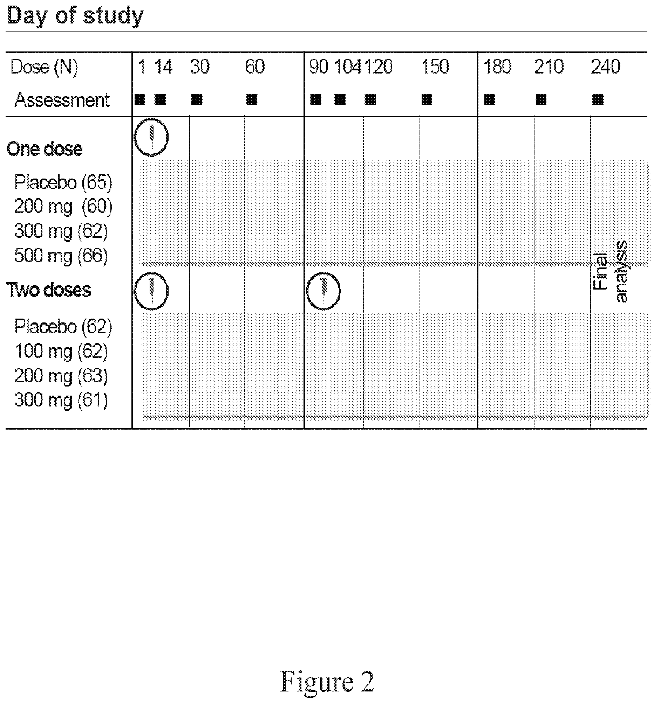

A summary of the study design is shown in Figure 2. On Day 1, all eligible patients were randomized and received the first subcutaneous administration of the RNAi agent or placebo. The RNAi agent was administered either as a single subcutaneous injection (doses: 200 mg, 300 mg), or two injections (dose: 500 mg). Patients randomized to receive a second dose received the second injection of RNAi agent or placebo at the Day 90.

The placebo was administered as either one or two subcutaneous injections of saline solution. The placebo volume was matched to the RNAi agent volume within each dose and injection regimen but not between injection regimens. For example, the placebo group for the 200 mg dose received 1.0 ml of placebo whereas the placebo group for the 300 mg dose received 1.5 mL of placebo.

The duration of the patients' involvement in the study was approximately 224 days which included screening, study drug administration, the course of single or multiple injections, and the follow-up period to Day 210. If additional follow-up was necessary the maximum duration of involvement was 374 days. End-of-study evaluations were conducted at Day 210 except for those patients whose LDL-C levels had not returned to within a 20 % deficit of starting level; these patients were evaluated at Day 210 and subsequently at follow-up visits occurring every 30 days until either Day 360, or LDL-C returned to within a 20 % deficit of starting level (whichever occurred first).

For the single dose groups (one or two injections on Day 1), patients followed the following schedule:
(a) screening: Day -14 to -1
(b) randomization, initiation of study drug: Day 1
(c) treatment phase: Day 1
(d) follow-up:
   (i) follow-up: Days 2 to 210; EOS on Day 210
   (ii) additional follow-up (for patients in whom LDL-C levels have not returned to >80 % of baseline values; patients will return each month for follow-up until this level has been reached or until Day 360): Days 240, 270, 300, 330, and 360.

For the two-dose groups (one injection each on Day 1 and Day 90), patients followed the following schedule:
(a) screening: Day -14 to -1
(b) randomization, initiation of study drug: Day 1
(c) treatment phase: Day 1 to Day 90
(d) follow-up:
   (i) follow-up: Days 91 to 210; EOS on Day 210
   (ii) additional follow-up (for patients in whom LDL-C levels have not returned to >80 % of baseline values; patients will return each month for follow-up until this level has been reached or until Day 360): Days 240, 270, 300, 330, and 360.

The primary endpoint was evaluated by determining the percentage change in LDL-C from baseline to Day 180.

The secondary endpoints were evaluated by determining the (i) percentage change in LDL-C from baseline to Day 90; (ii) percentage change in LDL-C from baseline to Days 14, 30, 60, 120, 150, and 210; (iii) proportion of patients in each group with LDL-C greater than 80 % of the baseline value at Day 180 and Day 210; (iv) duration of time on treatment for patients to return to 80 % of baseline or greater LDL-C or PCSK9 protein; (v) individual responsiveness defined as the number of patients reaching on treatment LDL-C levels of < 25 mg/dl, < 50 mg/dl, < 70 mg/dl, and < 100 mg/dl at Days 90, 120, and 180; (vi) proportion of patients in each group with greater or equal to 50 % LDL-C reduction from baseline at Days 90, 120, and 180; (vii) percentage change in PCSK9 levels from baseline to Days 14, 30, 60, 90, 104, 120, 150, 180, and 210; (viii) percentage change in other lipids, lipoproteins, apolipoproteins, from baseline at each subsequent visit to Day 210; and (ix) proportion of patients in each group who attain global lipid modification targets for their level of ASCVD risk.

Efficacy assessments included measuring the effects of the RNAi agent on levels of LDL-C lipids and lipoproteins including total cholesterol, triglycerides, HDL-C, non-HDL-C, VLDL-C, Apo-A1, Apo-B, Lp(a), CRP, and PCSK9.

Adverse events, serious adverse events, vital signs, clinical laboratory values (hematology, coagulation testing, chemistry, and urinalysis), and electrocardiograms (ECGs) was collected at specified visits through the EOS visit (Day 210). Adverse events, serious adverse events, and clinical laboratory values continued to be assessed during the additional monthly follow-up visits (for patients in whom LDL-C levels have not returned to > 80 % of baseline values). Cardiovascular events were reported as adverse events for the compilation of information on cardiovascular events such as cardiovascular death, non-fatal myocardial infarction, major coronary events (CHD death, resuscitated cardiac arrest, non-fatal myocardial infarction), ischemic stroke, and hemorrhagic stroke.

In addition, anti-drug antibodies were evaluated for the RNAi agent. Formation of anti-drug antibodies was assessed on Day 1 (prior to and four hours after the injection) and on Days 30, 60, 90, 120, 150, 180 (Days 150 and 180 only in patients who received a second dose of the RNAi agent), and 210 or until any anti-drug antibody response became negative within the study duration. For patients in whom LDL-C levels had not returned to > 80 % of baseline values, formation of anti-drug antibodies was assessed either when LDL-C had returned to normal limits or at the 1-year follow-up visit.

The independent Data Monitoring Committee (DMC) reviewed safety data beginning after the first 40 patients received the first injection of the RNAi agent or placebo and completed the Day 14 follow-up visit. Thereafter the DMC reviewed safety data every two months until the end of the trial.

The primary end point was analyzed as the least-squares mean percentage change from baseline to Day 180. This was calculated with a repeated-measurement linear-effects model, which included study group, baseline value, scheduled follow-up visit, and the interaction of study group with scheduled visit. The analysis was performed with the use of the PROC MIXED procedure in SAS software with an auto-regressive variance structure that incorporates treatment at each visit as fixed effects and patients as random effects. For both the primary and secondary end points, P values were adjusted for multiple comparisons with the use of Dunnett's test for comparison among the six RNAi agent groups and the placebo comparator groups. Separate analyses were performed for each dosing strategy-that is, a single dose and two doses. The type I error significance level was 0.05 for a two-sided test.

All patients who received at least one dose of the RNAi agent or placebo were included in the safety analysis (safety population). The prespecified modified intention-to-treat population was defined as all randomly assigned patients who received at least one dose of study agent and for whom both the baseline and the 180-day follow-up LDL-C level measurements were available. An intention-to-treat analysis was performed with the use of imputation for patients with missing data.

Time-course data are presented as means with 95% confidence intervals. Variation in responses among patients is depicted graphically with waterfall plots. Analyses were performed with SAS software, versions 9.2 and higher (SAS Institute).

### Results

The baseline demographics and history, shown in Tables 1 and 2, demonstrate that the patients were well balanced. At study, 73 % of patients were receiving statin therapy, and 31 % were on ezetimibe.

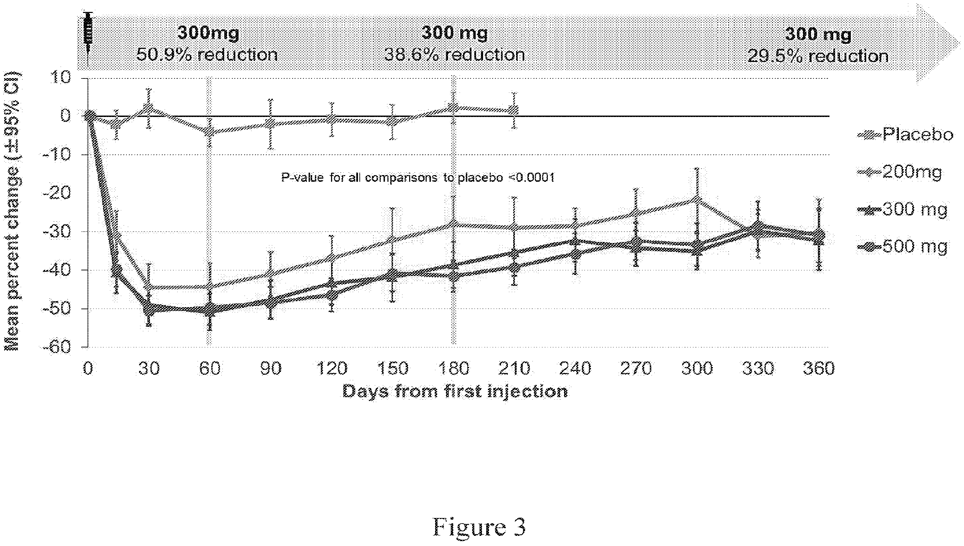

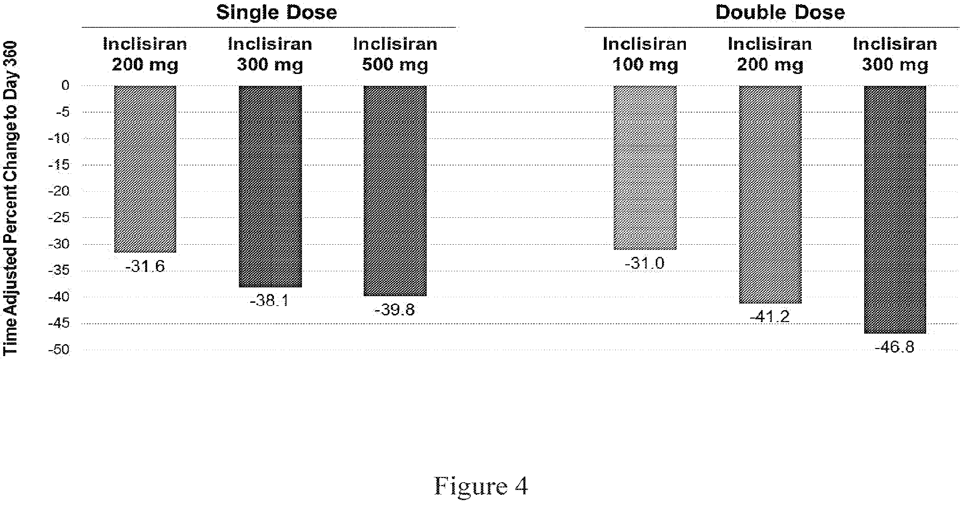

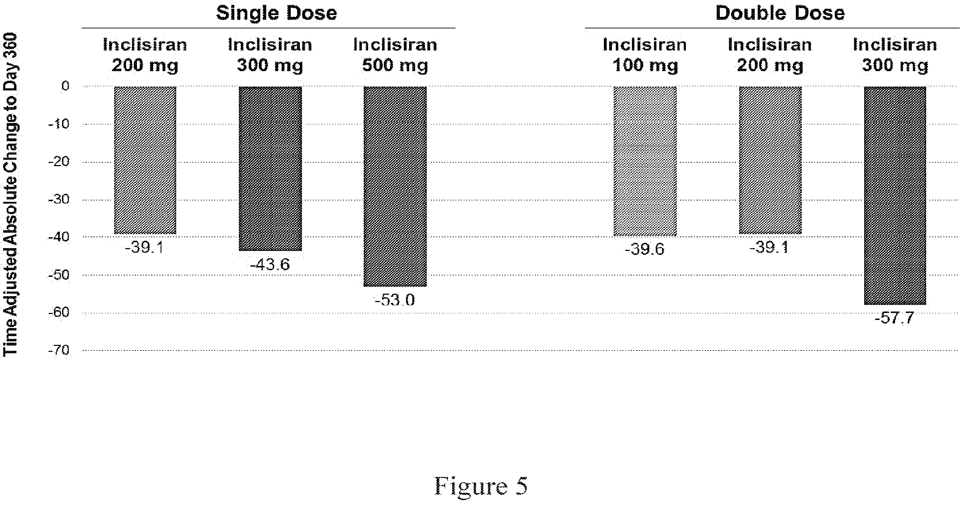

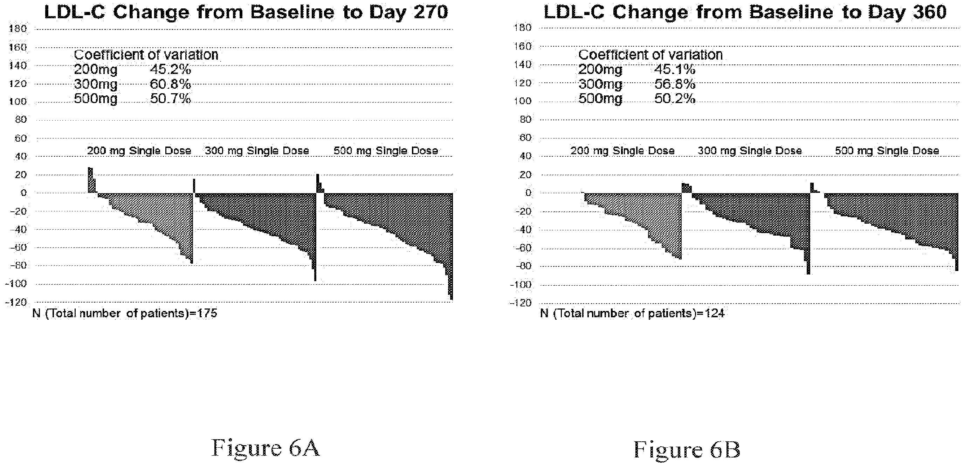

Single administration of the RNAi agent on Day 1 across all doses resulted in mean reductions in LDL-C ranging between 44.5 % and 51.5% below baseline (see Figures 3 and 4) at Day 30, with a nadir at approximately Day 60. Single administration of the 200-mg dose of the RNAi agent lowered LDL-C by over 40 % below baseline at Days 30, 60, and 90, and below 25 % through Day 270, as shown in Figure 3. Single administration of the 300-mg dose and 500-mg dose of the RNAi agent lowered LDL-C by over 40 % below baseline from Day 15 through Day 150, and below 30 % through Day 270 (Figure 3). For each of the RNAi agent dose strengths, LDL-C remained over 20 % below baseline through Day 360 (Figure 3). The least-squares mean reductions were significantly greater after a single dose of the RNAi agent (27.9 to 41.9 % reduction) than in association with placebo (2.1 % increase) (P<0.001 for all doses) (Table 4). The mean reduction in LDL-C from baseline across the 200-mg, 300-mg, and 500-mg doses ranged between 26 % and 34.3 % at Day 270, and between 30.2 % and 32.2 % at Day 360. Further, the time-adjusted percent change in LDL-C between baseline and Day 360 (i.e., the mean of the percent change of LDL-C at each timepoint that LDL-C was measured between baseline and Day 360) was -31.6 %, -38.1 %, and -39.8 % for the 200-mg, 300-mg, and 500-mg doses, respectively (see Figure 4). The time-adjusted absolute change in LDL-C between baseline and Day 360 (i.e., the mean of the absolute change of LDL-C at each timepoint that LDL-C was measured between baseline and Day 360) was -39.1 mg/dl, -43.6 mg/dl, and -53.0 mg/dl for the 200-mg, 300-mg, and 500-mg doses, respectively (see Figure 5). Turning to individual patient responses, the coefficient of variation of change in LDL-C among individual patients dosed with 200 mg, 300 mg, and 500 mg was 45.2 %, 60.8 %, and 50.7 %, respectively, from baseline to Day 270, and was 45.1 %, 56.8 %, and 50.2 %, respectively, from baseline to Day 360 (see Figures 6A-6B).

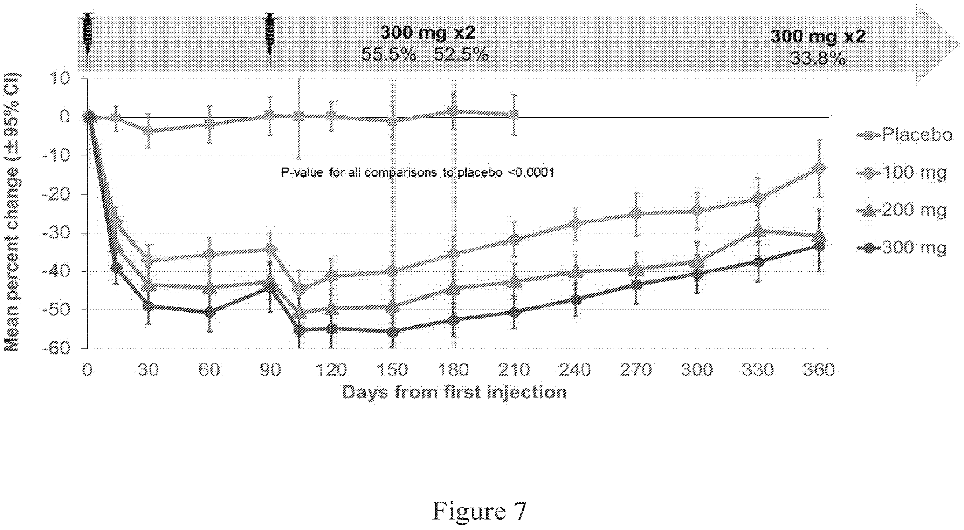

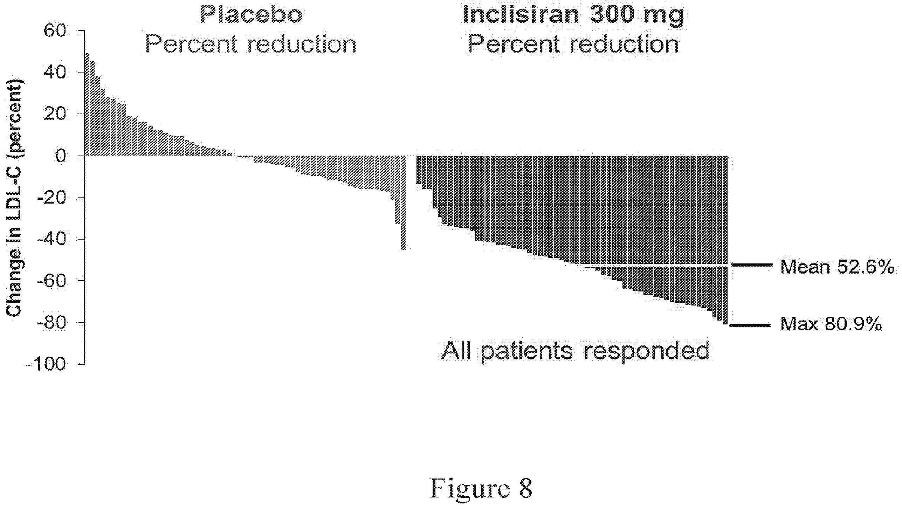

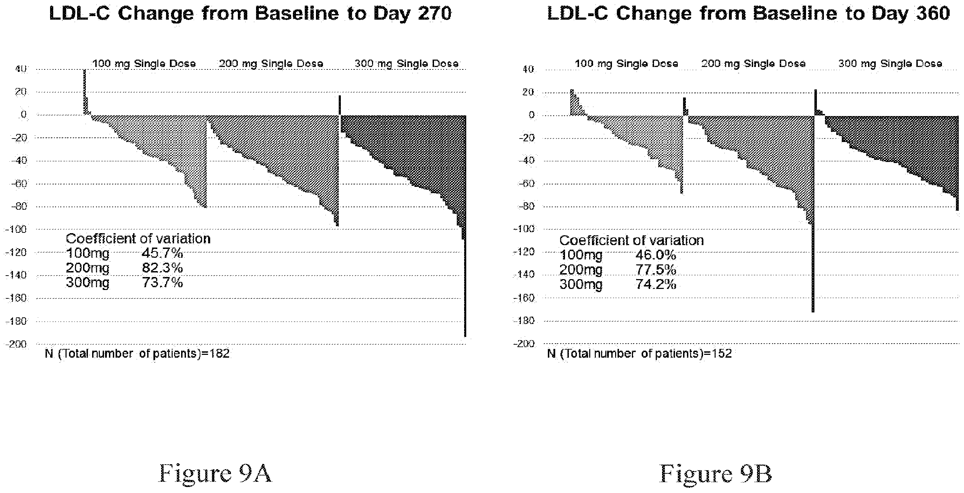

Administration of a second dose of the RNAi agent at Day 90 further lowered LDL-C as compared to baseline. Across all doses, the mean reductions in LDL-C ranged between 34.2 % and 44.1 % on Day 90, and between 41.1 % and 54.6 % on Day 120 (see Figure 7). The differences between the two-dose regimens and placebo were significant: at Day 180, the least-squares mean reductions in LDL-C levels from baseline among patients who received two doses of the RNAi agent ranged from 35.5 % to 52.6 %, whereas the placebo group had an increase from baseline of 1.8 % (P<0.001 for all comparisons vs. placebo) (see Table 4). The second administration of the 100-mg dose and the 200-mg dose of the RNAi agent maintained LDL-C at over 30 % and 40 %, respectively, below baseline through Day 210 (see Figure 7). The second administration of the 300-mg dose maintained LDL-C at over 50 % below baseline from Day 120 to Day 210 (Figure 7). The mean reduction in LDL-C from baseline across the 100-mg, 200-mg, and 300-mg doses ranged between 25.6 % and 43.4 % at Day 270, and between 13.3 % (100-mg dose) and 33.3 % (300-mg dose) at Day 360. In addition, the time-adjusted percent change in LDL-C between baseline and Day 360 was -31.0 %, -41.2 %, and -46.8 % for the 200-mg, 300-mg, and 500-mg doses, respectively (see Figure 4). The time-adjusted absolute change in LDL-C between baseline and Day 360 was -39.6 mg/dl, -39.1 mg/dl, and -57.7 mg/dl for the 200-mg, 300-mg, and 500-mg doses, respectively (see Figure 5). Individual patient responses are shown in Figures 8 and 9. At Day 180, the mean reduction in LDL-C from baseline was 52.6 %, and the maximum was 81 % (see Figure 8). The coefficient of variation of change in LDL-C among patients dosed with 100 mg, 200 mg, and 300 mg was 45.7 %, 82.3 %, and 73.7 %, respectively, from baseline to Day 270, and was 46.0 %, 77.5 %, and 74.2 %, respectively, from baseline to Day 360 (see Figures 9A-9B).

Among patients who received placebo against a background of the maximum possible dose of a statin, there was considerable variation at Day 180 in the changes in LDL cholesterol levels from baseline (mean [±SD] absolute difference, -0.7±25.6 mg per deciliter [-0.02±0.66 mmol per liter]) (see Figure 10A). In contrast, all patients who received two 300-mg doses of the RNAi agent had a decline in LDL-C level at Day 180 (mean absolute change in LDL cholesterol level, -64.2±20.7 mg per deciliter [-1.66±0.54 mmol per liter]) (see Figure 10B), and 54% of the patients had a reduction of 50 % or more. In this RNAi agent dose group, 5 %, 48 %, and 66 % of the patients had LDL-C levels at Day 180 of less than 25 mg per deciliter (0.65 mmol per liter), less than 50 mg per deciliter (1.3 mmol per liter), and less than 70 mg per deciliter (1.8 mmol per liter), respectively. At Day 240, the individual cholesterol levels remained lower than at baseline in the same patient group (see Figure 10C).

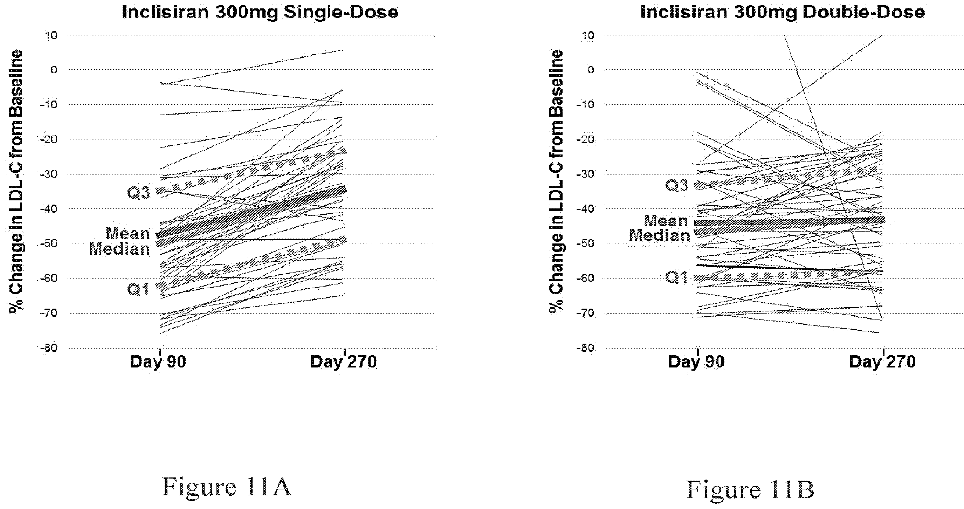

A comparison of individual patient data between the administration of a single dose of 300 mg and a double-dose of 300 mg suggests that administration of 300 mg RNAi agent on Day 1, Day 90, Day 270, and every 6 months thereafter should reduce within-person variability and provide sustained reductions in LDL-C of around 50 % (see Figures 11A-11B).

There were also significant reductions in levels of non-HDL-C and Apo-B, and no significant change in levels of high-sensitivity C-reactive protein among patients randomly assigned to receive the RNAi agent. The percentage changes from baseline for additional lipid measures are shown in Tables 3-5.

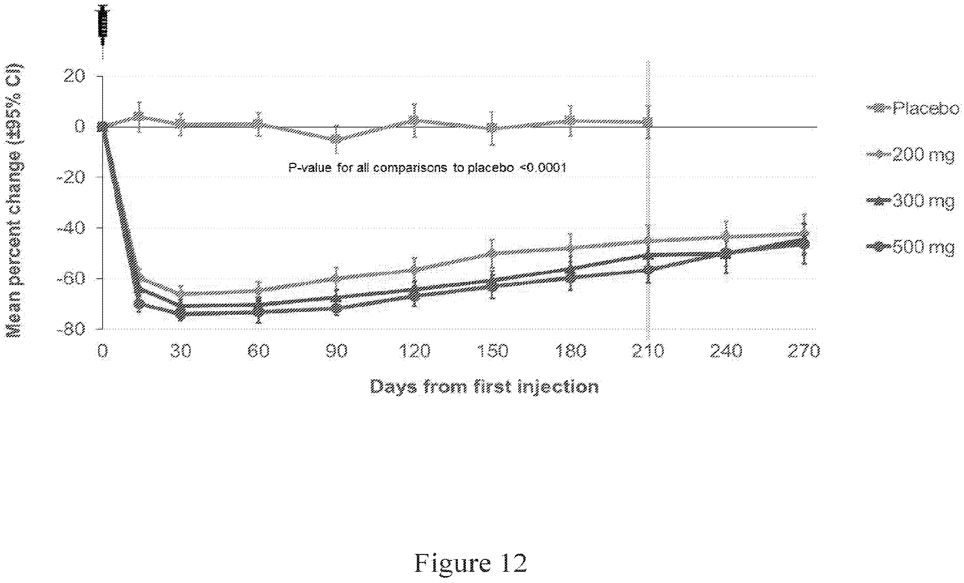

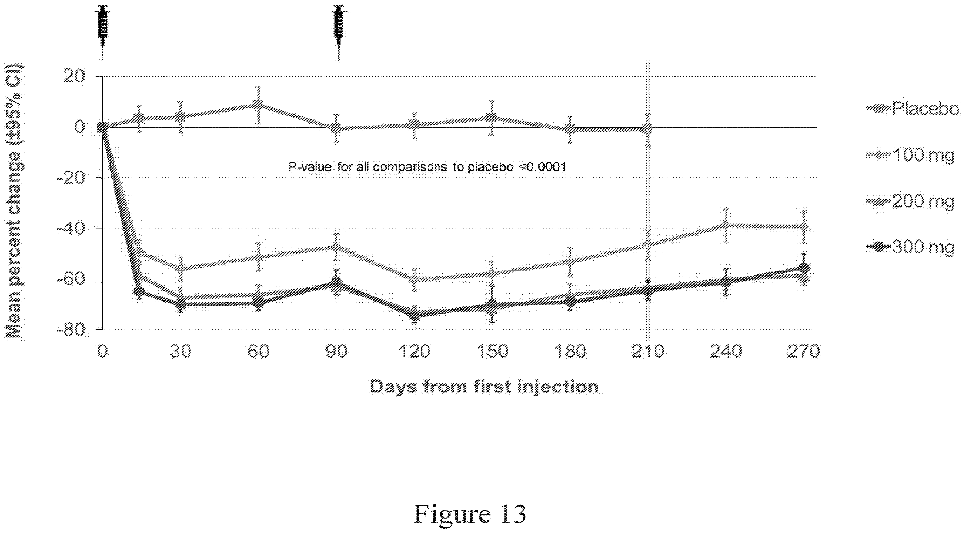

As shown in Figures 12 and 13, after the RNAi agent was administered on Day 1, PCSK9 levels were reduced from baseline levels by a mean of 59.6 % and 68.7 % across the range of RNAi agent doses from 100 mg to 500 mg. At Day 30, PCSK9 levels were further reduced to between 66.2 % and 74.0 % below baseline levels, and similar reductions occurred at Day 60 and Day 90. Among patients who received a single administration of the RNAi agent, the mean reductions in PCSK9 levels at Day 180 ranged between 47.9 % and 59.3 % (P<0.001 for each dose vs. placebo) (see Table 4). For patients receiving a single dose of 300-mg or 500-mg, PCSK9 levels remained over 60 % below baseline through Day 150. In comparison, among the patients who received two doses of the RNAi agent, further reductions in PCSK9 levels were observed after the second dose. At Day 90, these patients had reductions of 47.0 % to 62.8 %, and at Day 120, they had reductions of 60.4 % to 74.5 %. At Day 180, the mean reductions from baseline in PCSK9 levels among patients who received two doses of the RNAi agent ranged between 53.2 % and 69.1 % (P<0.001 for each dose vs. placebo) (see Table 4). For patients receiving two doses of 200-mg or 300-mg, PCSK9 levels remained over 60 % below baseline through Day 240. In association with both the single-dose and two-dose RNAi agent dosing regimens, the reductions in PCSK9 levels at Day 270 were greater than about 40 % (see Figures 12 and 13).

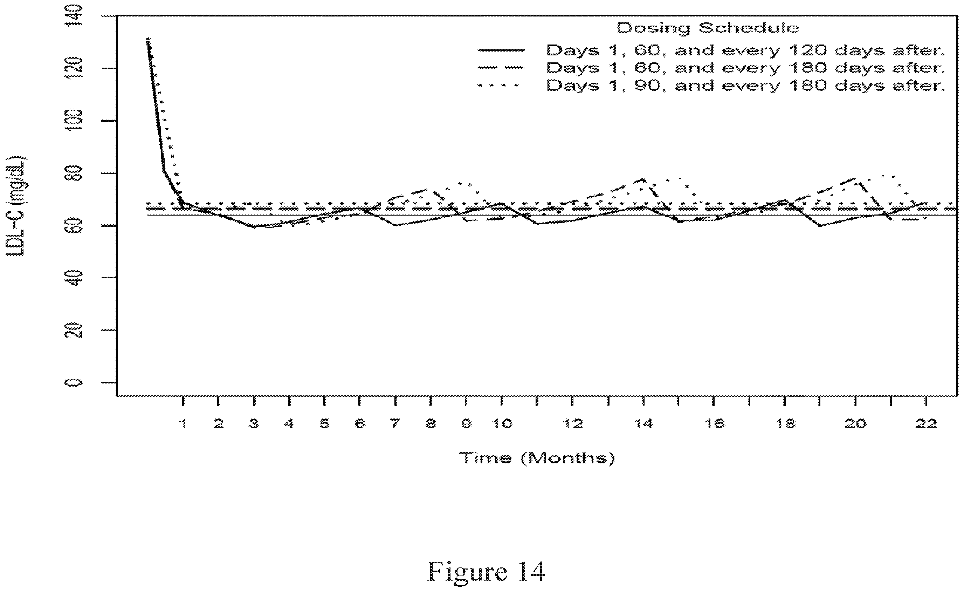

A model was developed and validated to project LDL-C levels over time when the RNAi agent is administered at 300 mg twice or thrice annually. The model was validated with minimal difference between observed and expected data to Day 210. Outcomes at Day 270 were correctly predicted by the pharmacodynamic model using data from patients with follow up to Day 210. Figure 14 shows the modeled LDL-C levels through 22 months.

At Day 210, adverse events were reported in 76 % of the patients who received the RNAi agent and in 76 % of the patients who received placebo (Table 7; see also Table 6). Most of these events (95 %) were mild or moderate in severity (grade 1 or 2). The incidence of serious adverse events was 11 % among patients who received the RNAi agent and 8 % among patients who received placebo. Two patients discontinued participation in the trial because of adverse events: one because of a herpes zoster infection (placebo group) and the other because of influenza or nasopharyngitis (two-dose 100-mg RNAi agent group). The most common adverse events (occurring in >2 % of patients) were myalgia, headache, fatigue, nasopharyngitis, back pain, hypertension, diarrhea, and dizziness, and the incidences of these events did not differ significantly between groups receiving the RNAi agent and those receiving placebo.

Injection-site reactions occurred in 4 % of the patients who received a single dose and in 7 % of the patients who received two doses (after one or both doses) of the RNAi agent (combined rate, 5 %); injection-site reactions occurred in no patients assigned to placebo (Table 7; see also Tables 8 and 9).

Two patients had increased levels of hepatic aspartate aminotransferase (>3 times the upper limit of the normal range), one in the single-dose placebo group and one in the single-dose 300-mg RNAi agent group; the patient in the 300-mg RNAi agent group also had elevations in hepatic alanine aminotransferase levels. Two additional patients (one in the two-dose 100-mg the RNAi agent group and one in the two-dose 300-mg RNAi agent group) also had elevations in alanine aminotransferase levels. All aminotransferase elevations were transient. There were no increases in bilirubin levels that occurred in association with the RNAi agent or placebo among patients who had normal levels of bilirubin at baseline, and no case met the definition of Hy's law, which states that a patient is at high risk for a fatal drug-induced liver injury if given a medication that causes hepatocellular injury (not cholestatic injury) with jaundice. One patient was positive for antidrug antibodies before the first injection; no other cases of antidrug antibody were reported (see Table 10).

There were no addition safety findings in follow up from Day 210 to Day 360 (see Table 11).

Two deaths occurred late in the trial. The first occurred in a patient who had been randomly assigned to the single-dose 500-mg RNAi agent group and who had long-standing vasculopathy and frequent angina. He had a witnessed cardiac arrest and died at 104 days. The second death occurred in a man in the two-dose 200-mg RNAi agent group who had a thoracic aortic aneurysm repaired percutaneously after study entry and in whom a fistula and sepsis subsequently developed; he died 198 days into the trial.

### Conclusion

These results showed that the RNAi agent reduced LDL-C significantly in patients over multiple months. Administration of the RNAi agent reduced Lp(a) and total cholesterol among other lipid parameters, and increased HDL-C. The RNAi agent showed a dose-response effect for PCSK9, LDL-C, non-HDL-C, and Apo-B. LDL-C variability within individual patients was practically eliminated, and there was a sustained effect between infrequent administrations.

In addition, administration of the RNAi agent was well tolerated by the patients and was associated with no major safety issues. The TEAEs were infrequent and mild to moderate, and there was little injection burden.

Overall, the RNAi agent offers an opportunity to improve patient adherence.

**Table 1. Baseline demographics and clinical characteristics.***

| | | | **Single Dose RNAi Agent vs. Placebo** | | | | **Two-Dose RNAi Agent vs. Placebo** | | | |
|---|---|---|---|---|---|---|---|---|---|---|
| | | | **Placebo** | **200 mg** | **300 mg** | **500 mg** | **Placebo** | **100 mg** | **200 mg** | **300 mg** |
| | | | **n = 65** | **n = 60** | **n = 61** | **n = 65** | **n = 62** | **n = 61** | **n = 62** | **n = 61** |
| **Age** | | years | 62.0±11.4 | 63.9±10.8 | 63.9±12.8 | 62.1±12.5 | 62.8±10.3 | 65.2±9.4 | 62.3±10.9 | 64.1±9.4 |
| **Race** | | n (%) | | | | | | | | |
| | **White** | | 59 (92)^{a} | 53 (90)^{b} | 55 (90) | 62 (95) | 58 (94) | 56 (92) | 60 (97) | 58 (95) |
| | **Other** | | 5 (8)^{a} | 6 (10)^{b} | 6 (10) | 3 (5) | 4 (6) | 5 (8) | 2(3) | 3 (5) |
| **Male gender** | | n (%) | 42 (65) | 39 (65) | 41 (67) | 46 (71) | 33 (53) | 38 (62) | 39 (63) | 45 (74) |
| **BMI** | | kg/m² | 30.1±5.1 | 28.2±5.5 | 28.1±4.2 | 27.9±4.2 | 29.2±4.8 | 29.2±6.0 | 30.5±5.0 | 29.2±6.7 |
| **Smokers, current^{c}** | | n (%) | 7 (10.8) | 9 (15.0) | 11 (18.0) | 8 (12.3) | 8 (12.9) | 7 (11.5) | 9 (14.5) | 7 (11.5) |
| **Hypertension** | | n (%) | 43 (66.2) | 38 (63.3) | 42 (68.9) | 38 (59.4) | 44 (72.1) | 45 (73.8) | 47 (75.8) | 43 (70.5) |
| **Prior ACSVD** | | n (%) | 45 (69) | 43 (72) | 48 (77) | 36 (55) | 46 (74) | 43 (69) | 41 (65) | 43 (70) |
| **Familial hypercholesterolemia** | | n (%) | 1(2) | 6 (10) | 2(3) | 7 (11) | 3 (5) | 3 (5) | 3 (5) | 3 (5) |
| **Diabetes mellitus** | | n (%) | 15 (23) | 12 (20) | 11 (18) | 11(17) | 14 (23) | 20 (33) | 21 (34) | 14 (23) |
| **Primary prevention** | | n (%) | 12(18) | 7 (12) | 7 (11) | 11(17) | 6 (10) | 6 (10) | 9 (14) | 7 (11) |
| **Any lipid modification treatment^{d}** | | n (%) | 50 (78) | 52 (87) | 50 (83) | 48 (80) | 51 (84) | 48 (81) | 49 (82) | 50 (85) |
| **Statin treatment** | | n (%) | 45 (70) | 50 (83) | 45 (75) | 39 (65) | 47 (77) | 42 (71) | 40 (67) | 43 (73) |
| **High intensity statin treatment** | | n (%) | 26 (41) | 31 (52) | 23 (38) | 20 (33) | 22 (36) | 28 (48) | 23 (38) | 20 (34) |
| **Exetemibe^{d}** | | n (%) | 21 (33) | 23 (38) | 17 (28) | 21 (35) | 17 (28) | 18 (31) | 20 (33) | 15 (25) |
| **Total cholesterol** | | mg/dl^{e} | 207.7±59.0 | 200.0±49.4 | 201.4±47.8 | 218.3±52.8 | 208.4±54.7 | 207.7±62.8 | 219.1±84.9 | 221.7±65.5 |
| **LDL-C** | | mg/dl^{e} | 128.5±51.3 | 122.8±35.9 | 117.8±40.5^{b} | 136.9±45.3 | 125.2±44.3^{b} | 128.5±49.5^{f} | 138.8±76.9^{b} | 131.3±60.3^{f} |
| **Non-HDL-C** | | mg/dl^{e} | 157.8±55.2 | 149.9±44.7 | 150.4±49.0 | 169.2±53.3 | 157.1±53.7 | 160.9±63.7 | 170.5±85.3 | 165.4±61.0 |
| **HDL-C** | | mg/dl^{e} | 49.9±13.6 | 50.0±11.7 | 51.0±13.3 | 49.1±15.4 | 51.2±16.1 | 46.8±14.0 | 48.6±13.0 | 47.4±13.6 |
| **Triglyceride** | | mg/dl^{e} | | | | | | | | |
| | **Median (IQR)^{e}** | | 125 (95-170) | 115 (84-149) | 134 (92-179) | 130 (94-193) | 137 (103-187) | 126 (91-198) | 127 (90-200) | 132 (105-185) |
| **VLDL-C** | | mg/dl^{e} | 23.9±18.6 | 27.1±19.7 | 31.5±19.6 | 32.4±19.2 | 30.8±17.0^{b} | 33.7±22.9^{f} | 31.7±19.8^{b} | 32.8±16.0^{f} |
| **Apo-B** | | mg/dl^{e} | 102.4±29.6 | 100.7±23.6 | 99.2±27.8 | 109.7±28.4 | 104.6±31.5 | 107.6±36.3 | 108.3±45.4 | 107.4±32.1 |
| **Apo-A1** | | mg/dl^{e} | 149.7±27.0 | 150.6±24.5 | 153.6±24.1 | 149.7±28.1 | 154.4±30.1 | 145.0±27.1 | 147.3±23.0 | 146.9±26.1 |
| **Lipoprotein(a)** | | nmol/dl^{e} | 27 (8-121)^{g} | 42 (11-129) | 35 (17-141) | 28 (12-149) | 50 (11-154) | 33 (12-128) | 36 (7-144) | 49 (12-161) |
| **PCSK9** | | ng/ml^{e} | 404.7±131.3 | 460.3±142.5 | 408.9±115.2 | 416.7±143.7 | 431.3±132.3 | 394.2±128.9^{h} | 437.4±141.8ⁱ | 416.3±127.3ⁱ |
| **High sensitivity C-reactive protein** | | mg/dl^{e} | | | | | | | | |
| | **Median (IQR)^{e}** | | 1.6 (0.7-3.1) | 1.0 (0.5-2.0) | 1.6 (0.7-3.5) | 1.9 (0.9-3.4) | 1.6 (0.8-4.4) | 1.3 (0.5-2.6) | 1.6 (0.7-3.0) | 1.8 (0.7-3.8) |

| | | | | | | | | | | |
|---|---|---|---|---|---|---|---|---|---|---|
| *plus-minus values are mean±SD BMI = body mass index; LDL-C = low-density lipoprotein cholesterol; HDL-C = high-density lipoprotein cholesterol; VLDL-C = very low-density lipoprotein cholesterol; Apo-B = apolipoprotein B; Apo-A1 = apolipoprotein A1; PSCK9 = proprotein convertase subtilisin/kexin type 9 ^{a}n = 64 ^{b}n = 59 ^{c}within past 30 days ^{d}mITT population (single dose: placebo, n=64; 200 mg, 300 mg and 500 mg, n=60; two doses: placebo, n=61; 100 mg and 300 mg, n= 59; 200 mg, n=60) ^{e}mITT population screening measurement (single dose: placebo, n=64; 200 mg, 300 mg and 500 mg, n=60; two doses: placebo, n=61; 100 mg, 200 mg, 300 mg, n= 59) ^{f}n=56 ^{g}n=63 ^{h}n=58 ⁱn=60 ^{j}n=57 | | | | | | | | | | |

**Table 2. Baseline demographics and clinical characteristics (with patients receiving the RNAi agent pooled).**

| | | **Single Dose RNAi Agent vs. Placebo** | | **Two-Dose RNAi Agent vs. Placebo** | |
|---|---|---|---|---|---|
| | | **Placebo** | **RNAi Agent** | **Placebo** | **RNAi Agent** |
| | | **n = 65** | **n = 186** | **n = 62** | **n = 184** |
| **Age** | Mean years | 62 | 63 | 63 | 64 |
| **Male gender** | % | 64.6 | 67.7 | 53.2 | 66.3 |
| **Prior ACSVD** | % | 69.2 | 67.9 | 74.2 | 68.3 |
| **Statin treatment** | % | 70.3 | 74.4 | 77.0 | 70.2 |
| **LDL-C** | Mean mg/dl | 128.5 | 125.9 | 125.2 | 133.0 |
| **Non-HDL-C** | Mean mg/dl | 157.8 | 156.5 | 157.1 | 165.6 |
| **Apo-B** | Mean mg/dl | 102.4 | 103.2 | 104.6 | 107.7 |
| **Lipoprotein(a)** | Median nmol/dl | 27.0 | 34.0 | 50.5 | 40.0 |
| **PCSK9** | Mean ng/ml | 404.7 | 428.7 | 431.3 | 416.2 |

**Table 3. Efficacy of the RNAi Agent on Lipid Parameters at Day 90 After One Dose, shown as percent change from baseline.¹**

| | | **Placebo** | **RNAi Agent** | | | |
|---|---|---|---|---|---|---|
| | | | **100 mg** | **200 mg** | **300 mg** | **500 mg** |
| | | **n = 124** | **n = 61** | **n = 122** | **n = 122** | **n = 65** |
| **Total Cholesterol** | mean (SD) | -0.5 % (16) | -22 % (12) | -26 % (14) | -28 % (15) | -30 % (11) |
| **Triglyceride** | median | 4 % (32) | -1 % (20) | -6 % (30) | -5 % (25) | -3 % (32) |
| **HDL-C** | mean (SD) | -2 % (14) | 5 % (11) | 8 % (12) | 9 % (16) | 8 % (15) |
| **Non-HDL-C** | mean (SD) | -0.2 % (20) | -30 % (13) | -37 % (18) | -40 % (19) | -42 % (15) |
| **Ap₀-B** | mean (SD) | -2 % (16) | -28 % (12) | -34 % (15) | -37 % (16) | -40 % (13) |
| **Lp(a)** | median | -0.4 % (31) | -18 % (19) | -21 % (23) | -14 % (114) | -25 % (27) |

| | | | | | | |
|---|---|---|---|---|---|---|
| ¹Includes patients with baseline and Day 90 measurement for all parameters HDL-C = high-density lipoprotein cholesterol Apo-B = apolipoprotein B Lp(a) = lipoprotein(a) | | | | | | |

**Table 4. Efficacy of the RNAi Agent on Lipid Parameters at Day 180, shown as percent change from baseline. ***

| | **Single Dose RNAi Agent vs. Placebo** | | | | **Two-Dose RNAi Agent vs. Placebo** | | | |
|---|---|---|---|---|---|---|---|---|
| | **Placebo** | **200 mg** | **300 mg** | **500 mg** | **Placebo** | **100 mg** | **200 mg** | **300 mg** |
| | **n = 64** | **n = 60** | **n = 60** | **n = 60** | **n = 61** | **n = 59** | **n = 60** | **n = 59** |
| **LDL-C^{a}** | 2.1 (-2.9 to 7.2) | -27.9 (-33.1 to 22.7)^{b} | -38.4 (-43.6 to -33.2)^{b} | -41.9 (-47.2 to -36.7)^{b} | 1.8 (-2.6 to 6.3) | -35.5 (-40.0 to -31.0)^{b} | -44.9 (-49.3 to -40.4)^{b} | -52.6 (-57.1 to -48.1)^{b} |
| **Total cholesterol** | 1.8±12.1 | -17.6±19.0^{b} | -23.7±15.7^{b} | -26.6±10.7^{b} | 0.7±12.3 | -22.4±12.4^{b} | -26.8±13.0^{b} | -33.2±11.3^{b} |
| **Non-HDL-C** | 1.5±16.7 | -25.1±26.2^{b} | -35.2±20.2^{b} | -36.9±14.0^{b} | 1.3±16.9 | -31.7±15.1^{b} | -38.9±16.8^{b} | -46.0±14.6^{b} |
| **HDL-C** | 3.8±15.6 | 4.4±14.8 | 8.8±11.1^{c} | 6.9±14.0 | 0.5±12.5 | 7.6±12.2^{c} | 10.3±15.3^{b} | 8.6±14.9^{d} |
| **Triglyceride^{e}** | 6.4 (-15.9 to 21.9) | 1.1 (-18.5 to 17.8) | -12.8 (-27.8 to 7.8)^{d} | -12.2 (-25.6 to 7.7)^{c} | -3.0 (-17.2 to 22.6) | -6.3 (-17.6 to 10.9) | 0.7 (-22.4 to 11.3) | -14.2 (-26.4 to 5.4)^{c} |
| **VLDL-C^{e}** | 2.4 (-30.7 to 30.5) | -11.6 (-35.8 to 23.3) | -23.8 (-43.0 to -6.4)^{d} | -14.6 (-34.8 to 3.5)^{c} | 2.7 (-20.0 to 26.7) | -16.4 (-31.3 to 0)^{d} | -21.2 (-38.5 to 13.2) | -16.0 (-38.2 to 9.1)^{d} |
| **Ap₀-B** | 1.7±14.7 | -22.9±21.0^{b} | -30.8±18.0^{b} | -33.1±12.7^{b} | 0.9±13.0 | -27.8±13.4^{b} | -35.0±15.8^{b} | -40.9±14.8^{b} |
| **Apo-A1** | 3.6±10.6 | 2.9±9.3 | 3.8±8.9 | 4.1±10.9 | 0.8±8.3 | 5.5±10.6 | 8.6±11.5^{d} | 6.2±11.9 |
| **Lipoprotein(a)^{e}** | 0.5 (-13.9 to 14.8) | -14.3 (-29.5 to -3.5) | -14.3 (-25.4 to -5.6) | -18.2 (-35 to -1.6) | 0.0 (-10.0 to 12.4) | -14.9 (-26.6 to -1.9) | -17.3 (-31.9 to -7.7) | -25.6 (-38.5 to -15.2) |
| **PCSK9** | 2.2±23.4 | -47.9±21.0^{b} | -56.0±19.2^{b} | -59.3±18.0^{b} | -1.2±20.7 | -53.2±20.9^{b} | -66.2±15.6^{b} | -69.1±12.1^{b} |
| **High sensitivity C-reactive protein^{e}** | -5.3 (-40.8 to 28.4) | 7.1 (-30.7 to 70.9) | -16.2 (-45.8 to 50) | -19.8 (-50 to 32.7) | -20 (-50 to 30) | -12.5 (-42.9 to 29.4) | -16.3 (-34.6 to 24.3) | -16.7 (-50.9 to -33.3)^{c} |

| | | | | | | | | |
|---|---|---|---|---|---|---|---|---|
| * Plus-minus values are mean ±SD. Data are presented for the modified intention-to-treat population, which consisted of all patients who underwent randomization, who received at least one dose of study agent, and in whom both the baseline and Day 180 LDL-C measurements were available. The numbers of patients who were excluded because of missing data at Day 180 were as follows: for the single-dose regimens, 1 in the placebo group (2 %), 2 in the 300-mg RNAi agent group (3 %), and 6 in the 500-mg RNAi agent group (9 %); for the two-dose regimens, 1 in the placebo group (2 %), 3 in the 100-mg RNAi agent group (5 %), 3 in the 200-mg RNAi agent group (5 %), and 6 in the 300-mg RNAi agent group (10 %). The P values for the treatment-by-visit interaction are 0.11 for the single-dose regimen and 0.07 for the two-dose regimen. This analysis is based on modeling percentage change from baseline with treatment, planned visits through Day 180, the treatment-by-visit interaction, and baseline LDL-C level. "Data are least-squares means and 95% confidence intervals. ^{b}The comparison with the change in the placebo group indicated a significant difference (P<0.001 by Dunnett's adjusted test). ^{c}The comparison with the change in the placebo group indicated a significant difference (P<0.05 by Dunnett's adjusted test). ^{d}The comparison with the change in the placebo group indicated a significant difference (P<0.01 by Dunnett's adjusted test). ^{e}Data are medians and interquartile ranges. LDL-C = low-density lipoprotein cholesterol; HDL-C = high-density lipoprotein cholesterol; VLDL-C = very low-density lipoprotein cholesterol; Apo-B = apolipoprotein B; Apo-A1 = apolipoprotein A1; PSCK9 = proprotein convertase subtilisin/kexin type 9 | | | | | | | | |

**Table 5. Efficacy of the RNAi Agent on Lipid Parameters at Day 210 After Two Dose, shown as change from baseline.**

| | | **Placebo** | **RNAi Agent** | | |
|---|---|---|---|---|---|
| | | | **100 mg** | **200 mg** | **300 mg** |
| | | **n = 61** | **n = 59** | **n = 60** | **n = 59** |
| **Total Cholesterol** | mean (SD) | 0 % (14) | -21 % (11) | -26 % (12) | -31 % (13) |
| **Triglyceride** | Median | 2 % | -10 % | -10 % | -9 % |
| **HDL-C** | mean (SD) | -1 % (15) | 7 % (15) | 11 % (14) | 9 % (15) |
| **Non-HDL-C** | mean (SD) | 0 % (18) | -29 % (14) | -38 % (15) | -43 % (17) |
| **Ap₀-B** | mean (SD) | 1 % (14) | -24 % (13) | -33 % (14) | -39 % (15) |
| **Lp(a)** | median | -4 % | -18 % | -20 % | -26 % |

| | | | | | |
|---|---|---|---|---|---|
| HDL-C = high-density lipoprotein cholesterol; Apo-B = apolipoprotein B; Lp(a) = lipoprotein(a); | | | | | |

**Table 6. Treatment Emergent Adverse Events (TEAE)¹ and Other Safety Parameters through Day 90.**

| | **Placebo** | **RNAi Agent** | | | | |
|---|---|---|---|---|---|---|
| | | **Pooled** | **100 mg** | **200 mg** | **300 mg** | **500 mg** |
| | **n = 127** | **n = 370** | **n = 61** | **n = 122** | **n = 122** | **n = 65** |
| **Any TEAE** | 69 (54 %) | 198 (54 %) | 38 (62 %) | 64 (52 %) | 68 (56 %) | 28 (43 %) |
| **Serious** | 5 (4 %) | 22 (6 %) | 8 (13 %) | 6 (5 %) | 6 (5 %) | 2 (3 %) |
| **Severe** | 5 (4 %) | 12 (3 %) | 3 (5 %) | 3 (2 %) | 4 (3 %) | 2 (3 %) |
| **Related** | 24 (19 %) | 67 (18 %) | 11 (18 %) | 20 (16 %) | 27 (22 %) | 9 (14 %) |
| **Death** | 0 (0 %) | 1 (0.3 %) | 0 (0 %) | 0 (0 %) | 0 (0 %) | 1 (1.5 %) |
| **ALT > 3x ULN** | 0 | 1 (0.3 %) | 0 | 0 | 1 (0.8 %) | 0 |
| **AST > 3x ULN** | 0 | 1 (0.3 %) | 0 | 0 | 1 (0.8 %) | 0 |
| **ALP > 2x ULN** | 0 | 3 (0.8 %) | 1 (1.6 %) | 0 | 2 (1.6 %)² | 0 |
| **Bilirubin > 2x ULN³** | 0 | 0 | 0 | 0 | 0 | 0 |
| **CK> 5x ULN** | 0 | 2 (0.6 %) | 0 | 1 (0.8 %)⁴ | 1 (0.8 %) | 0 |
| **Myalgia** | 6 (4.7 %) | 21 (5.7 %) | 5 (8.2 %) | 7 (5.7 %) | 8 (6.6 %) | 1 (1.5 %) |

| | | | | | | |
|---|---|---|---|---|---|---|
| ¹Crossing above threshold for significance at any time after randomization regardless of baseline ²One patient was above ULN at baseline 3No patient met the criteria for Hy's law ⁴Patient's CK > 3x ULN at baseline ALT = alanine aminotransferase; AST = aspartate aminotransferase; ALP = alkaline aminotransferase; ULN = upper limit normal; CK = creatine kinase | | | | | | |

**Table 7. Treatment Emergent Adverse Events (TEAE)¹ and Other Safety Parameters through Day 210.***

| | | **Single Dose RNAi Agent vs. Placebo** | | | | **Two-Dose RNAi Agent vs. Placebo** | | | |
|---|---|---|---|---|---|---|---|---|---|
| | | **Placebo** | **200 mg** | **300 mg** | **500 mg** | **Placebo** | **100 mg** | **200 mg** | **300 mg** |
| | | **n = 65** | **n = 60** | **n = 61** | **n = 65** | **n = 62** | **n = 61** | **n = 62** | **n = 61** |
| **Any event that occurred during treatment** | | 46 (71) | 47 (78) | 44 (72) | 49 (75) | 50 (81) | 48 (79) | 47 (76) | 47 (77) |
| | **Serious** | 3 (5) | 6 (10) | 5 (8) | 6 (9) | 6 (10) | 11 (18) | 6 (10) | 7 (11) |
| | **Severe** | 2 (3) | 2 (3) | 4 (7) | 5 (8) | 7 (11) | 5 (8) | 6 (10) | 8 (13) |
| | **Death** | 0 | 0 | 0 | 1 (2)^{a} | 0 | 0 | 1 (2)^{b} | 0 |
| **Injection Site Reaction^{c}** | | 0 | 2 (3) | 2 (3) | 3 (5) | 0 | 3 (5) | 5 (8) | 4 (7) |
| **ALT level > 3x ULN** | | 0 | 0 | 1 (2)^{d} | 0 | 0 | 1(2) | 0 | 1 (2) |
| **AST level > 3x ULN** | | 1 (2) | 0 | 1 (2)^{d} | 0 | 0 | 0 | 0 | 0 |
| **ALP level > 2x ULN** | | 0 | 0 | 2 (3) | 1 (2) | 0 | 2 (3) | 0 | 0 |
| **Bilirubin level > 2x ULN³** | | 1 (2) | 0 | 0 | 0 | 0 | 1(2) | 0 | 0 |
| **CK level > 5x ULN** | | 1 (2)^{e} | 0 | 3 (5)^{f} | 0 | 0 | 0 | 2 (3)^{g} | 0 |
| **Myalgia** | | 3 (5) | 2 (3) | 5 (8) | 3 (5) | 3 (5) | 7 (11) | 5 (8) | 5 (8) |
| **Glycated hemoglobin^{h}** | | -0.2±7.0 | -0.1±6.1 | -0.1±3.3 | 0±6.9 | 0.3±13.7 | 0.6±10.4 | 0.5±5.4 | -1.0±6.4 |
| **Plateletsⁱ** | | 1.7±12.0 | 4.8±12.6 | 0.7±12.5 | 1.4±15.6 | 5.6±20.3 | -0.1±10.9 | 4.3±17.5 | 0.7±15.6 |

| | | | | | | | | | |
|---|---|---|---|---|---|---|---|---|---|
| * Plus-minus values are mean ±SD. The numbers of patients completing follow-up to day 210 in each group were as follows: in the single-dose cohort: 63 patients in the placebo group, 60 in the 200-mg RNAi agent group, 61 in the 300-mg RNAi agent group, and 61 in the 500-mg RNAi agent group; in the two-dose cohort: 60 in the placebo group, 59 in the 100-mg RNAi agent group, 60 in the 200-mg RNAi agent group, and 59 in the 300-mg RNAi agent group. ^{a}The death was due to a myocardial infarction. ^{b}The death was due to sepsis and pneumonia after complications of surgery for aortic disease. ^{c}This category included rash, erythema, and pruritus. ^{d}The elevated ALT and AST levels were in the same patient. ^{e}The creatine kinase level in this patient was more than 73 times the upper limit of the normal range at baseline. ^{f}One patient had a creatine kinase level that was more than 8 times the upper limit of the normal range at baseline. ^{g}One patient had a creatine kinase level that was more than 4 times the upper limit of the normal range at baseline. ^{h}Glycated hemoglobin was measured in plasma and was assessed at day 180. ⁱPlatelet count was measured in whole blood. ALP = alkaline phosphatase; ALT = alanine aminotransferase; AST = aspartate aminotransferase; ULN = upper limit of normal; CK = creatine kinase | | | | | | | | | |

**Table 8. Injection Site Treatment Emergent Adverse Event^{1,2} After the First Injection to Day 90.**

| | **Placebo** | **RNAi Agent** | | | | |
|---|---|---|---|---|---|---|
| | | **Pooled** | **100 mg** | **200 mg** | **300 mg** | **500 mg** |
| | **n = 127** | **n = 370** | **n = 61** | **n = 122** | **n = 122** | **n = 65** |
| **Injection site erythema** | 0 | 4 (1.1 %) | 0 | 2 (1.6 %) | 1 (0.8 %) | 1 (1.5 %) |
| **Injection site pruritus** | 0 | 1 (0.3 %) | 0 | 0 | 1 (0.8 %) | 0 |
| **Injection site rash** | 0 | 0 | 0 | 0 | 0 | 0 |
| **Injection site reaction** | 0 | 7 (1.9 %) | 1 (1.6%) | 1 (0.8 %) | 3 (2.5 %) | 2 (3.1 %) |
| **Total (observed any time)** | 0 | 12 (3.2 %) | 1 (1.6 %) | 3 (2.5 %) | 5 (4.1 %) | 3 (4.6 %) |
| **Total (observed > 4 hours)** | 0 | 9 (2.4 %) | 1 (1.6 %) | 3 (2.5 %) | 4 (3.3 %) | 1 (1.5 %) |

| | | | | | | |
|---|---|---|---|---|---|---|
| ¹Number of patients with adverse event classified by preferred term-each patient is counted only once ²Pre-defined histaminic/allergic type adverse events | | | | | | |

**Table 9. Injection Site Treatment Emergent Adverse Event^{1,2} After Two Injections from Day 90 to Day 210.**

| | **Placebo** | **RNAi Agent** | | | |
|---|---|---|---|---|---|
| | | **Pooled** | **100 mg** | **200 mg** | **300 mg** |
| | **n = 60** | **n = 178** | **n = 57** | **n = 61** | **n = 60** |
| **Injection site erythema** | 0 | 1 (0.6 %) | 0 | 1 (1.6 %) | 0 |
| **Injection site pruritus** | 0 | 4 (2.2 %) | 1 (1.8 %) | 3 (4.9%) | 0 |
| **Injection site rash** | 0 | 3 (1.7 %) | 2 (3.5 %) | 0 | 1 (1.7 %) |
| **Injection site reaction** | 0 | 2 (1.1 %) | 0 | 2 (3.3 %) | 0 |
| **Total (observed any time)** | 1 (1.7%) | 13 (7.3 %) | 4 (7.0 %) | 3 (4.9%) | 6 (10.0 %) |

| | | | | | |
|---|---|---|---|---|---|
| ¹Number of patients with adverse event classified by preferred term-each patient is counted only once ²Pre-defined histaminic/allergic type adverse events | | | | | |

**Table 10. Anti-drug Antibodies After Two Doses to Day 210.**

| | **Placebo** | **RNAi Agent** | | | |
|---|---|---|---|---|---|
| | | **Total RNAi Agent** | **100 mg** | **200 mg** | **300 mg** |
| | **n = 62** | **n = 184** | **n = 61** | **n = 62** | **n = 61** |
| **Baseline** | 0 | 1 (0.5 %) | 0 | 1 (1.6 %)¹ | 0 |
| **Day 90** | 0 | 1 (0.5 %) | 0 | 1 (1.6 %)¹ | 0 |
| **Day 210** | 0 | 0 | 0 | 0 | 0 |

| | | | | | |
|---|---|---|---|---|---|
| ¹"Borderline positive sample with pre-existing / cross reactive antibodies" | | | | | |

**Table 11. Treatment Emergent Adverse Events (TEAE) and Other Safety Parameters through Day 360.**

| | **Single Dose RNAi Agent vs. Placebo** | | **Two-Dose RNAi Agent vs. Placebo** | |
|---|---|---|---|---|
| | **Placebo** | **RNAi Agent** | **Placebo** | **RNAi Agent** |
| | **n = 65** | **n = 186** | **n = 62** | **N=184** |
| **Any TEAE** | 51 (78.5 %) | 155 (81.3 %) | 51 (82.3 %) | 153 (83.2 %) |
| **Serious TEAE** | 3 (4.6 %) | 30 (16.1 %) | 7(11.3 %) | 31 (16.8 %) |
| **Severe TEAE** | 2 (3.1 %) | 18 (9.7 %) | 7 (11.3 %) | 22 (12.0 %) |
| **Related TEAE** | 12 (18.5%) | 39 (21.0 %) | 19 (30.6 %) | 52 (28.3 %) |
| **Adverse Event Discontinuation** | 0 | 0 | 1 (1.6 %) | 1 (0.5 %) |
| **Injection Site Reaction** | 0 | 7 (3.8 %) | 0 | 12 (6.5 %) |

| | | | | |
|---|---|---|---|---|
| One dose starting regimen: nasopharyngitis, myalgia, back pain, cough, arthralgia, headache Two dose starting regimen: myalgia, headache, diarrhea, nasopharyngitis, arthralgia, back pain | | | | |

### SEQUENCE LISTING

SEQ ID NO: 1
   5'- ACAAAAGCAAAACAGGUCUAGAA - 3'
SEQ ID NO: 2
   5'- CUAGACCUGUTUUGCUUUUGU - 3'
SEQ ID NO: 3
   5'- asCfsaAfAfAfgCfaAfaAfcAfgGfuCfuagsasa - 3', in which in which a, g, c and u are 2'-O-methyl (2'-OMe) A, G, C, or U; Af, Gf, Cf or Uf are 2'-fluoro A, G, C or U; dT is 2'-deoxythymidine; and s is a phosphorothioate linkage
SEQ ID NO: 4
   5'- csusagacCfuGfudTuugcuuuugu - 3', in which a, g, c and u are 2'-O-methyl (2'-OMe) A, G, C, or U; Af, Gf, Cf or Uf are 2'-fluoro A, G, C or U; dT is 2'-deoxythymidine; and s is a phosphorothioate linkage

## Claims

1. An interfering ribonucleic acid (RNAi) agent for use in (i) the treatment of an atherosclerotic cardiovascular disease (ASCVD), an ASCVD risk equivalent, an elevated risk for cardiovascular disease (CVD), heterozygous familial hypercholesterolemia, or homozygous familial hypercholesterolemia, in a subject, or (ii) the prevention of a cardiovascular event, or the prevention of the development of an ASCVD, in a subject;
wherein the RNAi agent is a double-stranded ribonucleic acid comprising a sense strand and an antisense strand that forms a double-stranded region, the antisense strand comprising the nucleotide sequence of SEQ ID NO: 3, and the sense strand comprising the nucleotide sequence of SEQ ID NO: 4, wherein SEQ ID NO: 3 is 5'-asCfsaAfAfAfgCfaAfaAfcAfgGfuCfuagsasa-3', in which a, g, c, and u are 2'-O-methyl (2'-OMe) A, G, C, and U, respectively; Af, Gf, Cf, and Uf are 2'-fluoro A, G, C, and U, respectively; and s is a phosphorothioate linkage, and SEQ ID NO: 4 is 5'-csusagacCfuGfudTuugcuuuugu-3', in which a, g, c, and u are 2'-O-methyl (2'-OMe) A, G, C, and U, respectively; Af, Gf, Cf, and Uf are 2'-fluoro A, G, C, and U, respectively; dT is 2'-deoxythymidine; and s is a phosphorothioate linkage, and wherein the double-stranded ribonucleic acid has a covalently attached triantennary GalNAc ligand; and
wherein the RNAi agent is to be administered as a subcutaneous injection in a dosing regimen that comprises a loading phase followed by a maintenance phase, wherein the loading phase comprises administering the RNAi agent as at least two doses separated by a time interval, wherein the time interval is about 90 days.

2. The RNAi agent for use according to claim 1, wherein the use is for the treatment of atherosclerotic cardiovascular disease (ASCVD), an ASCVD risk equivalent, an elevated risk for cardiovascular disease, heterozygous familial hypercholesterolemia, or homozygous familial hypercholesterolemia, by lowering low-density lipoprotein cholesterol (LDL-C).

3. The RNAi agent for use according to claim 1 or 2, wherein the use is for the treatment of ASCVD or heterozygous familial hypercholesterolemia.

4. The RNAi agent for use according to claim 1, wherein the cardiovascular event is selected from nonfatal myocardial infarction, severe recurrent ischemia, stroke, and symptomatic pulmonary embolism.

5. The RNAi agent for use according to any one of claims 1-4, wherein the subject is on a diet.

6. The RNAi agent for use according to any one of claims 1-5, wherein the subject is being treated with a background lipid-lowering therapy.

7. The RNAi agent for use according to claim 6, wherein the background lipid-lowering therapy is a maximally tolerated statin therapy.

8. The RNAi agent for use according to any one of claims 1-7, wherein the subject is an adult human.

9. The RNAi agent for use according to any one of claims 1-8, wherein the subject requires additional lowering of LDL-C.

10. The RNAi agent for use according to any one of claims 1-9, wherein administration of the RNAi agent to the subject reduces the level of LDL-C by greater than about 20 % as compared to a baseline LDL-C level.

11. The RNAi agent for use according to claim 10, wherein the reduction in the level of LDL-C of greater than about 20 % as compared to the baseline level is maintained for 15 days or more after the RNAi agent is administered.

12. The RNAi agent for use according to any one of claims 1-11, wherein the at least two doses administered in the loading phase each comprise 100 mg to 500 mg of the RNAi agent.

13. The RNAi agent for use according to any one of claims 1-12, wherein the at least two doses administered in the loading phase each comprise 250 mg to 350 mg of the RNAi agent.

14. The RNAi agent for use according to any one of claims 1-13, wherein the at least two doses administered in the loading phase each comprise about 300 mg of the RNAi agent.

15. The RNAi agent for use according to any one of claims 1-14, wherein the maintenance phase comprises administering the RNAi agent as at least two doses separated by a regular time interval, wherein the regular time interval is between 3 and 9 months.

16. The RNAi agent for use according to claim 15, wherein the regular time interval separating the at least two doses administered in the maintenance phase is about 6 months.

17. The RNAi agent for use according to any one of claims 15-16, wherein the at least two doses administered in the maintenance phase each comprise 100 mg to 500 mg of the RNAi agent.

18. The RNAi agent for use according to any one of claims 15-17, wherein the at least two doses administered in the maintenance phase each comprise 250 mg to 350 mg of the RNAi agent.

19. The RNAi agent for use according to any one of claims 15-18, wherein the at least two doses administered in the maintenance phase each comprise about 300 mg of the RNAi agent.

20. The RNAi agent for use according to any one of claims 1-19, wherein the double-stranded ribonucleic acid comprises a ligand which is an N-acetylgalactosamine (GalNAc) derivative conjugated to the 3' end of the sense strand of the double-stranded ribonucleic acid.

21. The RNAi agent for use according to claim 20, wherein the RNAi agent is as depicted in the following schematic:

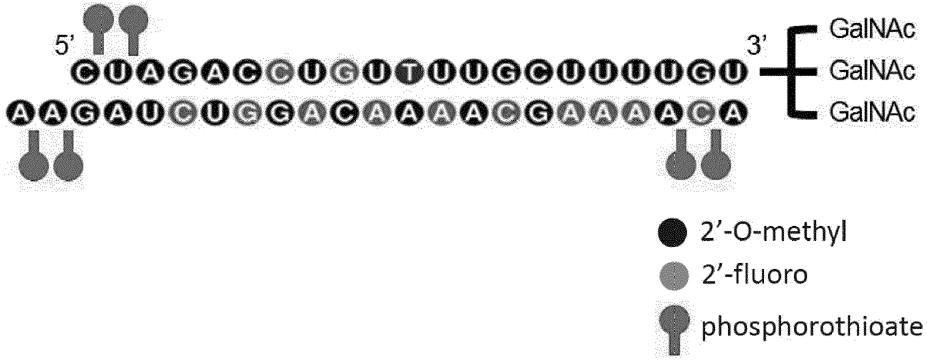
5'
C U A G A C C U C U T U U G C U U U U G U
3'
A A G A U C U G G A C A A A A C G A A A A C A
GalNAc
GalNAc
GalNAc
2'-O-methyl
2'-fluoro
phosphorothioate

22. The RNAi agent for use according to any one of claims 1-21, wherein the RNAi agent is formulated in a suitable pharmaceutical formulation at about 200 mg/ml such that administration of about 1.5 ml of the formulation to a subject provides a 300 mg fixed dose of the RNAi agent.

## Patentansprüche

1. Ein interferierende-Ribonukleinsäure(RNAi)-Mittel zur Verwendung in (i) der Behandlung einer atherosklerotischen kardiovaskulären Erkrankung (ASC VD), eines ASCVD-Risikoäquivalents, eines erhöhten Risikos für kardiovaskuläre Erkrankungen (CVD), von heterozygoter familiärer Hypercholesterinämie oder homozygoter familiärer Hypercholesterinämie, bei einem Subjekt oder (ii) der Verhinderung eines kardiovaskulären Ereignisses oder der Verhinderung der Entwicklung einer ASCVD bei einem Subjekt;
wobei das RNAi-Mittel eine doppelsträngige Ribonukleinsäure ist, umfassend einen Sense-Strang und einen Antisense-Strang, der eine doppelsträngige Region ausbildet, wobei der Antisense-Strang die Nukleotidsequenz von SEQ ID NO: 3 umfasst und der Sense-Strang die Nukleotidsequenz von SEQ ID NO: 4 umfasst, wobei SEQ ID NO: 3 5'-asCfsaAfAfAfgCfaAfaAfcAfgGfuCfuagsasa-3' ist, wobei a, g, c und u jeweils 2'-O-Methyl(2'-OMe)-A, -G, -C bzw. -U sind; Af, Gf, Cf und Uf 2'-Fluor-A, -G, -C bzw. -U sind; und s eine Phosphorthioatbindung ist und SEQ ID NO: 4 5'-csusagacCfuGfudTuugcuuuugu-3' ist, wobei a, g, c und u 2'-O-Methyl(2'-OMe)-A, -G, -C bzw. -U sind; Af, Gf, Cf und Uf 2'-Fluor-A, -G, -C bzw. -U sind; dT 2'-Desoxythymidin ist; und s eine Phosphorthioatbindung ist, und wobei die doppelsträngige Ribonukleinsäure einen kovalent gebundenen triantennären GalNAc-Liganden aufweist; und
wobei das RNAi-Mittel als subkutane Injektion in einem Dosierungsschema zu verabreichen ist, das eine Ladephase gefolgt von einer Erhaltungsphase umfasst, wobei die Ladephase das Verabreichen des RNAi-Mittels in mindestens zwei Dosen umfasst, die durch ein Zeitintervall getrennt sind, wobei das Zeitintervall etwa 90 Tage beträgt.

2. RNAi-Mittel zur Verwendung nach Anspruch 1, wobei die Verwendung zur Behandlung von atherosklerotischen kardiovaskulären Erkrankungen (ASCVD), eines ASCVD-Risikoäquivalents, eines erhöhten Risikos für kardiovaskuläre Erkrankungen, heterozygoter familiärer Hypercholesterinämie oder homozygoter familiärer Hypercholesterinämie durch Senkung des Spiegels von Low-Density-Lipoprotein-Cholesterol (LDL-C) ist.

3. RNAi-Mittel zur Verwendung nach Anspruch 1 oder 2, wobei die Verwendung zur Behandlung von ASCVD oder heterozygoter familiärer Hypercholesterinämie ist.

4. RNAi-Mittel zur Verwendung nach Anspruch 1, wobei das kardiovaskuläre Ereignis ausgewählt ist aus nichttödlichem Myokardinfarkt, schwerer wiederkehrender Ischämie, Schlaganfall und symptomatischer Lungenembolie.

5. RNAi-Mittel zur Verwendung nach einem der Ansprüche 1-4, wobei sich das Subjekt auf Diät befindet.

6. RNAi-Mittel zur Verwendung nach einem der Ansprüche 1-5, wobei das Subjekt mit einer lipidsenkenden Hintergrundtherapie behandelt wird.

7. RNAi-Mittel zur Verwendung nach Anspruch 6, wobei die lipidsenkende Hintergrundtherapie eine maximal verträgliche Statintherapie ist.

8. RNAi-Mittel zur Verwendung nach einem der Ansprüche 1-7, wobei das Subjekt ein erwachsener Mensch ist.

9. RNAi-Mittel zur Verwendung nach einem der Ansprüche 1-8, wobei das Subjekt eine zusätzliche Senkung von LDL-C benötigt.

10. RNAi-Mittel zur Verwendung nach einem der Ansprüche 1-9, wobei die Verabreichung des RNAi-Mittels an das Subjekt den LDL-C-Spiegel im Vergleich zu einem LDL-C-Ausgangswert um mehr als etwa 20 % senkt.

11. RNAi-Mittel zur Verwendung nach Anspruch 10, wobei die Verringerung des LDL-C-Spiegels um mehr als etwa 20 % im Vergleich zum Ausgangswert 15 Tage oder länger nach der Verabreichung des RNAi-Mittels aufrechterhalten wird.

12. RNAi-Mittel zur Verwendung nach einem der Ansprüche 1-11, wobei die mindestens zwei in der Ladephase verabreichten Dosen jeweils 100 mg bis 500 mg des RNAi-Mittels umfassen.

13. RNAi-Mittel zur Verwendung nach einem der Ansprüche 1-12, wobei die mindestens zwei in der Ladephase verabreichten Dosen jeweils 250 mg bis 350 mg des RNAi-Mittels umfassen.

14. RNAi-Mittel zur Verwendung nach einem der Ansprüche 1-13, wobei die mindestens zwei in der Ladephase verabreichten Dosen jeweils etwa 300 mg des RNAi-Mittels umfassen.

15. RNAi-Mittel zur Verwendung nach einem der Ansprüche 1-14, wobei die Erhaltungsphase das Verabreichen des RNAi-Mittels in mindestens zwei Dosen umfasst, getrennt durch ein regelmäßiges Zeitintervall, wobei das regelmäßige Zeitintervall zwischen 3 und 9 Monaten liegt.

16. RNAi-Mittel zur Verwendung nach Anspruch 15, wobei das regelmäßige Zeitintervall zwischen den mindestens zwei in der Erhaltungsphase verabreichten Dosen etwa 6 Monate beträgt.

17. RNAi-Mittel zur Verwendung nach einem der Ansprüche 15-16, wobei die mindestens zwei in der Erhaltungsphase verabreichten Dosen jeweils 100 mg bis 500 mg des RNAi-Mittels umfassen.

18. RNAi-Mittel zur Verwendung nach einem der Ansprüche 15-17, wobei die mindestens zwei in der Erhaltungsphase verabreichten Dosen jeweils 250 mg bis 350 mg des RNAi-Mittels umfassen.

19. RNAi-Mittel zur Verwendung nach einem der Ansprüche 15-18, wobei die mindestens zwei in der Erhaltungsphase verabreichten Dosen jeweils etwa 300 mg des RNAi-Mittels umfassen.

20. RNAi-Mittel zur Verwendung nach einem der Ansprüche 1-19, wobei die doppelsträngige Ribonukleinsäure einen Liganden umfasst, der ein N-Acetylgalactosamin(GalNAc)-Derivat ist, das an das 3'-Ende des Sense-Strangs der doppelsträngigen Ribonukleinsäure konjugiert ist.

21. RNAi-Mittel zur Verwendung nach Anspruch 20, wobei das RNAi-Mittel der Darstellung im folgenden Schema entspricht:

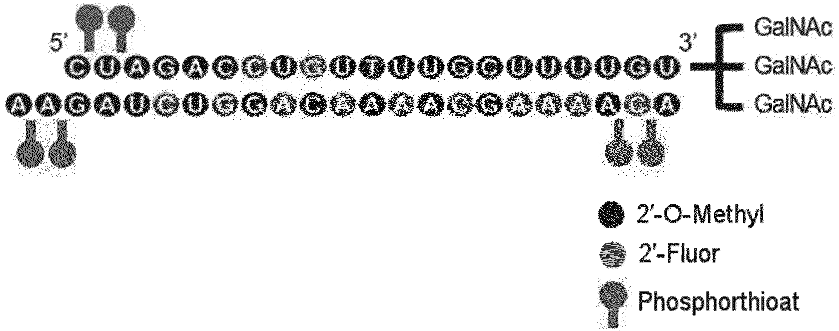
5'
C U A G A C C U G U T U U G C U U U U G U
3'
GalNAc
GalNAc
GalNAc
A A G A U C U G G A C A A A A C G A A A A C A
2'-O-Methyl
2'-Fluor
Phosphorthioat

22. RNAi-Mittel zur Verwendung nach einem der Ansprüche 1-21, wobei das RNAi-Mittel in einer geeigneten pharmazeutischen Formulierung mit etwa 200 mg/ml formuliert ist, sodass die Verabreichung von etwa 1,5 ml der Formulierung an ein Subjekt eine feste Dosis von 300 mg des RNAi-Mittels ergibt.

## Revendications

1. Agent d'acide ribonucléique interférent (ARNi) pour une utilisation dans (i) le traitement d'une maladie cardiovasculaire athérosclérotique (ASCVD), d'un équivalent de risque de ASCVD, d'un risque élevé pour une maladie cardiovasculaire (CVD), d'une hypercholestérolémie familiale hétérozygote ou d'une hypercholestérolémie familiale homozygote, chez un sujet, (ii) la prévention d'un événement cardiovasculaire, ou la prévention du développement d'une ASCVD, chez un sujet ;
l'agent d'ARNi étant un acide ribonucléique double brin comprenant un brin sens et un brin antisens qui forme une région à double brin, le brin antisens comprenant la séquence de nucléotides de la SEQ ID NO: 3, et le brin sens comprenant la séquence de nucléotides de la SEQ ID NO: 4, la SEQ ID NO: 3 étant 5'-asCfsaAfAfAfgCfaAfaAfcAfgGfuCfuagsasa-3', dans laquelle a, g, c, et u sont 2'-O-méthyle (2'-OMe) A, G, C, et U, respectivement ; Af, Gf, Cf, et Uf sont 2'-fluoro A, G, C, et U, respectivement ; et s est une liaison phosphorothioate, et SEQ ID NO: 4 est 5'-csusagacCfuGfudTuugcuuuugu-3', dans laquelle a, g, c, et u sont 2'-O-méthyle (2'-OMe) A, G, C, et U, respectivement ; Af, Gf, Cf, et Uf sont 2'-fluoro A, G, C, et U, respectivement ; dT est 2'-deoxythymidine ; et s est une liaison phosphorothioate, et l'acide ribonucléique double brin ayant un ligand GalNAc triantennaire fixé de manière covalente ; et
l'agent d'ARNi devant être administré comme injection sous cutanée dans un schéma posologique qui comprend une phase de charge suivie par une phase de maintenance, la phase de charge comprenant une administration de l'agent d'ARNi en tant qu'au moins deux doses séparées par un intervalle de temps, l'intervalle de temps étant d'environ 90 jours.

2. Agent d'ARNi pour une utilisation selon la revendication 1, l'utilisation étant pour le traitement d'une maladie cardiovasculaire athérosclérotique (ASCVD), d'un équivalent de risque de ASCVD, d'un risque élevé pour une maladie cardiovasculaire, d'une hypercholestérolémie familiale hétérozygote ou d'une hypercholestérolémie familiale homozygote, par un abaissement du cholestérol de lipoprotéines de basse densité (LDL-C).

3. Agent d'ARNi pour une utilisation selon la revendication 1 ou 2, l'utilisation étant pour le traitement d'ASCVD ou d'une hypercholestérolémie familiale hétérozygote.

4. Agent d'ARNi pour une utilisation selon la revendication 1, l'événement cardiovasculaire étant choisi parmi un infarctus du myocarde non fatal, une ischémie récurrente sévère, un accident vasculaire cérébral et une embolie pulmonaire asymptomatique.

5. Agent d'ARNi pour une utilisation selon l'une quelconque des revendications 1 à 4, le sujet suivant un régime.

6. Agent d'ARNi pour une utilisation selon l'une quelconque des revendications 1 à 5, le sujet étant traité avec une thérapie de fond d'abaissement de lipides.

7. Agent d'ARNi pour une utilisation selon la revendication 6, la thérapie de fond d'abaissement de lipides étant une thérapie par statines tolérées de manière maximale.

8. Agent d'ARNi pour une utilisation selon l'une quelconque des revendications 1 à 7, le sujet étant un humain adulte.

9. Agent d'ARNi pour une utilisation selon l'une quelconque des revendications 1 à 8, le sujet nécessitant un abaissement supplémentaire de LDL-C.

10. Agent d'ARNi pour une utilisation selon l'une quelconque des revendications 1 à 9, l'administration de l'agent d'ARNi au sujet réduisant le niveau de LDL-C de plus d'environ 20 % par comparaison avec un niveau de LDL-C de ligne de base.

11. Agent d'ARNi pour une utilisation selon la revendication 10, la réduction du niveau de LDL-C de plus d'environ 20 % par comparaison avec le niveau de ligne de base étant maintenue pendant 15 jours ou plus après que l'agent d'ARNi a été administré.

12. Agent d'ARNi pour une utilisation selon l'une quelconque des revendications 1 à 11, les au moins deux doses administrées dans la phase de charge comprenant chacune 100 mg à 500 mg de l'agent d'ARNi.

13. Agent d'ARNi pour une utilisation selon l'une quelconque des revendications 1 à 12, les au moins deux doses administrées dans la phase de charge comprenant chacune 250 mg à 350 mg de l'agent d'ARNi.

14. Agent d'ARNi pour une utilisation selon l'une quelconque des revendications 1 à 13, les au moins deux doses administrées dans la phase de charge comprenant chacune environ 300 mg de l'agent d'ARNi.

15. Agent d'ARNi pour une utilisation selon l'une quelconque des revendications 1 à 14, la phase de maintenance comprenant une administration de l'agent d'ARNi en tant qu'au moins deux doses séparées par un intervalle de temps régulier, l'intervalle de temps régulier étant compris entre 3 et 9 mois.

16. Agent d'ARNi pour une utilisation selon la revendication 15, l'intervalle de temps régulier séparant les au moins deux doses administrées dans la phase de maintenance étant d'environ 6 mois.

17. Agent d'ARNi pour une utilisation selon l'une quelconque des revendications 15 à 16, les au moins deux doses administrées dans la phase de maintenance comprenant chacune 100 mg à 500 mg de l'agent d'ARNi.

18. Agent d'ARNi pour une utilisation selon l'une quelconque des revendications 15 à 17, les au moins deux doses administrées dans la phase de maintenance comprenant chacune 250 mg à 350 mg de l'agent d'ARNi.

19. Agent d'ARNi pour une utilisation selon l'une quelconque des revendications 15 à 18, les au moins deux doses administrées dans la phase de maintenance comprenant chacune environ 300 mg de l'agent d'ARNi.

20. Agent d'ARNi pour une utilisation selon l'une quelconque des revendications 1 à 19, l'acide ribonucléique double brin comprenant un ligand qui est un dérivé de N-acétylgalactosamine (GalNAc) conjugué à l'extrémité 3' du brin sens de l'acide ribonucléique double brin.

21. Agent d'ARNi pour une utilisation selon la revendication 20, l'agent d'ARNi étant tel que décrit dans le schéma suivant :

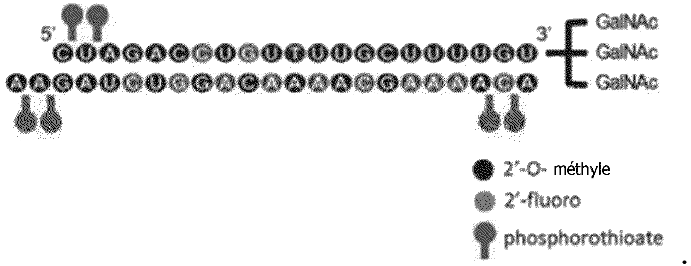
5' 3'
C U A G A C C U G U U U U G C U U U U G U
A A G A U C U G G A C A A A A C G A A A A C A
GalNAc
GalNAc
GalNAc
2'-O- méthyle
2'-fluoro
phosphorothioate

22. Agent d'ARNi pour une utilisation selon l'une quelconque des revendications 1 à 21, l'agent d'ARNi étant formulé dans une formulation pharmaceutique appropriée à raison d'environ 200 mg/ml de sorte qu'une administration d'environ 1,5 ml de la formulation à un sujet fournit une dose fixe de 300 mg de l'agent d'ARNi.
